(19) Europäisches Patentamt
European Patent Office
Office européen des brevets

(11) Publication number: 0 357 345
A2

# (12) EUROPEAN PATENT APPLICATION

(21) Application number: 89308618.1

(22) Date of filing: 24.08.89

(51) Int. Cl.5: C 07 D 495/04
A 01 N 47/36
//(C07D495/04,333:00,331:00)

(30) Priority: 29.08.88 US 237199 22.05.89 US 356053

(43) Date of publication of application:
07.03.90 Bulletin 90/10

(84) Designated Contracting States: ES GR

(71) Applicant: E.I. DU PONT DE NEMOURS AND COMPANY
1007 Market Street
Wilmington Delaware 19898 (US)

(72) Inventor: Amuti, Kofi Sam
5412 Valley Green Drive
Wilmington Delaware 19808 (US)

Cuomo, John
16 Hillcroft Road
Newark Delaware 19711 (US)

(74) Representative: Hildyard, Edward Martin et al
Frank B. Dehn & Co. European Patent Attorneys Imperial
House 15-19 Kingsway
London WC2B 6UZ (GB)

## (54) Herbicides for weed control in sugar beets, oilseed rape and cole crops.

(57) Control of weeds in crops, especially in sugar beets, oilseed rape, cole crops, lentils, flax and safflower is, accomplished with an effective amount of a compound of the formula:

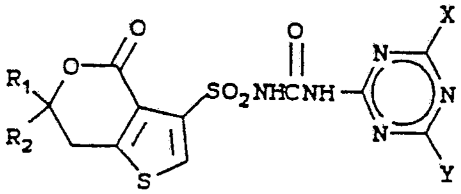

I

wherein
$R_1$ is H, $CH_3$ or $CH_2CH_3$;
$R_2$ is H or $CH_3$;
X is $OCH_3$ or $OCH_2CH_3$; and
Y is $NHCH_3$, $N(CH_3)_2$, $N(CH_3)OCH_3$, $OCH_3$, $OCH_2CH_3$, $OCH_2CF_3$, $CH_2CH_3$, $CH(CH_3)_2$ or cyclopropyl;
and their agriculturally suitable salts.

EP 0 357 345 A2

Description

## HERBICIDES FOR WEED CONTROL IN SUGAR BEETS, OILSEED RAPE AND COLE CROPS

### BACKGROUND OF THE INVENTION

U.S. 4,737,184, issued April 12, 1988, discloses, in part, herbicidal sulfonylureas of the formula

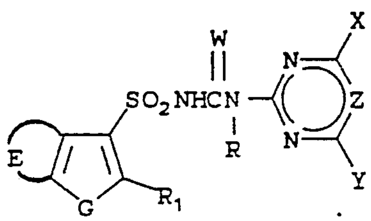

wherein
E is, inter alia,
a bridge of 3 or 4 atoms, which may be substituted or unsubstituted, containing 0-2 heteroatoms selected from the group consisting of oxygen, sulfur and nitrogen and also containing 1-4 atoms of carbon, said bridge together with two carbon attachment sites forming a partially saturated 5 to 6 membered carbocyclic or heterocyclic ring;
in the bridging group E, nitrogen may take the form of N or N-O, sulfur may take the form of S, SO or $SO_2$, and one of the atoms of carbon may be a carbonyl, thiocarbonyl or the cyclic 5 and 6 membered ketals thereof; when one of the bridging atoms is a substituted carbon, the substituent on said carbon includes, inter alia, H, $C_1$-$C_4$ alkyl, etc.;
G is O, S, NH or $NCH_3$;
$R_1$ is, inter alia, H, etc.;
X is, inter alia, $C_1$-$C_4$ alkoxy;
Y is, inter alia, $C_1$-$C_4$ alkyl, $C_1$-$C_4$ alkoxy, $C_1$-$C_4$ haloalkoxy, $C_1$-$C_3$ alkylamino, di-($C_1$-$C_3$-alkyl)amino, $C_3$-$C_5$ cycloalkyl, $N(OCH_3)CH_3$, etc.; and
Z is CH, N, $CCH_3$, $CC_2H_5$, CCl or CBr.

While this reference generically describes the compounds of the instant invention, it does not disclose their particular utility in sugar beets, oilseed rape, cole crops, lentils, flax and safflower.

### SUMMARY OF THE INVENTION

This invention comprises the novel use of compounds of Formula I to control undesired vegetation in sugar beet, oilseed rape, cole crop, lentil, flax and safflower plantings.

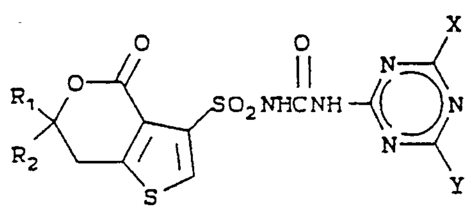

I

wherein
$R_1$ is H, $CH_3$ or $CH_2CH_3$;
$R_2$ is H or $CH_3$;
X is $OCH_3$ or $OCH_2CH_3$; and
Y is $NHCH_3$, $N(CH_3)_2$, $N(CH_3)OCH_3$, $OCH_3$, $OCH_2CH_3$, $OCH_2CF_3$, $CH_2CH_3$, $CH(CH_3)_2$ or cyclopropyl; and their agriculturally suitable salts.

This invention also comprises novel compounds of Formula I, wherein when $R_1$ and $R_2$ are H, and Y is $NHCH_3$, then X is $OCH_3$; their agriculturally suitable salts, agriculturally suitable compositions containing them, and their method-of-use as preemergence and/or postemergence herbicides or plant growth regulants.

#### Preferred Embodiments

Preferred for reasons of better weed control is the use of compounds of Formula I wherein at least one of $R_1$ and $R_2$ is H.

Specifically preferred for reasons of highest herbicidal activity, greatest safety to sugar beets, oilseed rape, cole crops, lentils, flax and/or safflower, and/or most favorable ease of synthesis is:
N-[[[4-(dimethylamino)-6-ethoxy-1,3,5-triazin-2-yl]amino]carbonyl]-6,7-dihydro-4-oxo-4H-thieno[3,2-c]pyran-3-sulfonamide.

Further Preferred is the use of the compound of Preferred 2 to control undesired vegetation in sugar beet, oilseed rape, cole crop, lentils, flax and safflower plantings.

## DETAILED DESCRIPTION OF THE INVENTION

### Synthesis

Many of the compounds of Formula I can be prepared by reaction of sulfonamides of Formula 2 with heterocyclic carbamates of Formula 3 in dioxane or acetonitrile at 20° to 80°C for periods of 1/2 to 24 hours in the presence of 1 equivalent of 1,8-diazabicyclo[5.4.0]undec-7-ene (DBU), as depicted in Equation 1. The resultant products are isolated by dilution of the reaction mixture with water, acidification and subsequent filtration. Heterocyclic carbamates of Formula 3 in turn are synthesized by reaction of heterocyclic amines of Formula 4 with hexamethyldisilazane; the resulting silylamines are then treated with phenyl chloroformate in petroleum ether at reflux. The carbamates may also be prepared by direct reaction of the heterocyclic amines with diphenyl carbonate or phenyl chloroformate in pyridine or with sodium hydride in dimethylformamide (DMF) at temperatures ranging from 20° to 80°C, as indicated in Equations 1a and 1b.

Equation 1

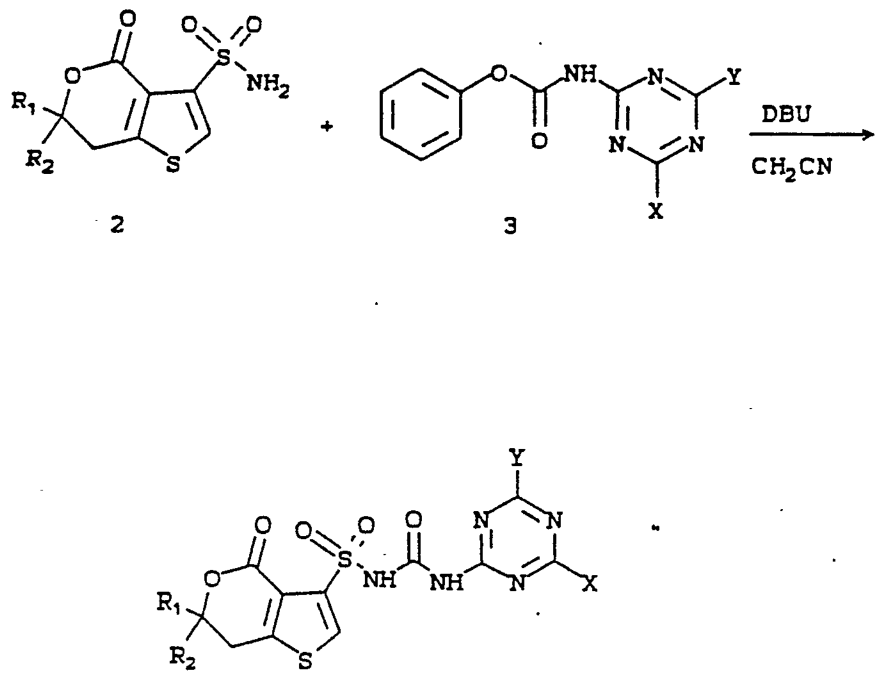


2 3

I

Equation 1a

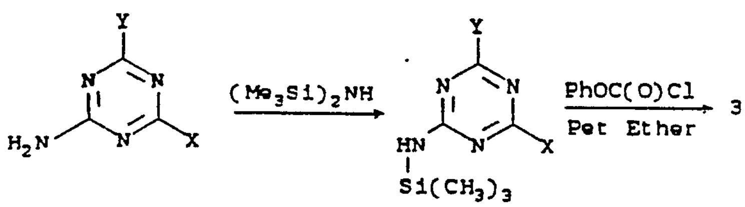


3

4 5

Equation 1b

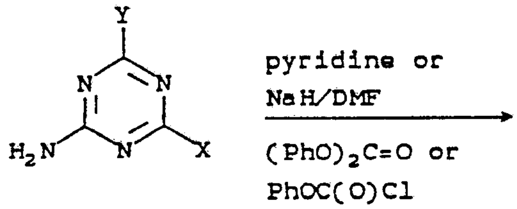


4

3

The intermediate thiophenelactonesulfonamides of Formula 2 may be prepared by the sequence of reactions shown in Equations 2 through 7.

3,4-Dibromothiophene can be transmetallated with n-butyllithium in ether at temperatures ranging from -100° to -70°C. The lithium anion can then be quenched with a disulfide, for example propyl disulfide, at temperatures ranging from -100° to -70°C. The resulting thioether 7 is isolated after standard aqueous workup and is in turn transmetallated with n-butyllithium in ether at temperatures ranging from -100° to -70°C and then quenched with carbon dioxide. The resulting acid 8 is extracted into aqueous sodium hydroxide solution, precipitated by acidification, filtered and dried.

Equation 2

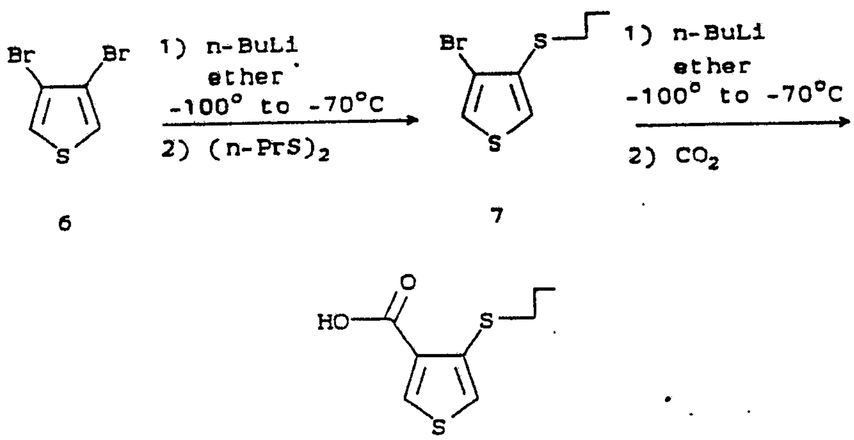


6

7

8

The acid 8 can then be treated with 2 equivalents of n-butyllithium in tetrahydrofuran (THF) solution at temperatures ranging from -80° to 20°C for 1 to 3 hours to form the dianion 9. This dianion is next treated with methyl iodide at temperatures ranging from -80° to 20°C. The product 10 is isolated by standard aqueous workup as depicted in Equation 3.

Equation 3

8

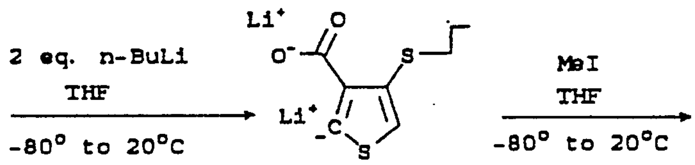


9

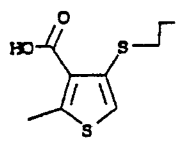

10

The methylated acid 10 can be converted to a dianion 11 by similar treatment with 2 equivalents of n-butyllithium in THF solution at temperatures ranging from -80° to 0°C for 1 to 3 hours. Reaction of 11 with ketones or aldehydes at temperatures ranging from -80° to 20°C followed by standard workup gives the alcohol 12a as shown in Equation 4.

Equation 4

10

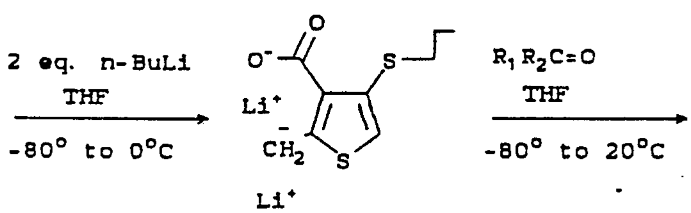


11

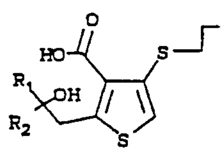

12a

The alcohol 12b where $R_1$ and $R_2$ = H can be prepared directly from dianion 9 by reaction with ethylene oxide in THF solution at temperatures ranging from -80° to 0°C, as depicted in Equation 5.

Equation 5

9 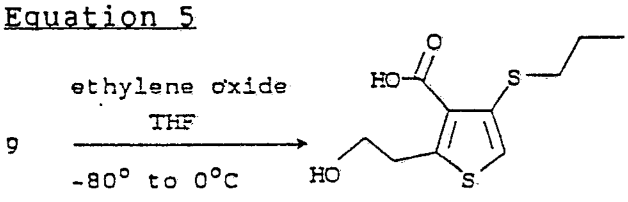


12b

The alcohols 12a or 12b can be cyclized to the lactone 13 by treatment with a catalytic amount of p-toluenesulfonic acid in benzene solution at reflux for 2 to 24 hours. Oxidative chlorination of the lactone 13 by standard methods known to one skilled in the art gives the sulfonyl chloride 14, as shown in Equation 6. In some cases it may be easier to first oxidize 13 to the sulfoxide 15 with m-chloroperbenzoic acid (MCPBA) in methylene chloride solution at temperatures ranging from -80° to 0°C prior to oxidative chlorination as depicted in Equation 6a.

Equation 6

12a or 12b 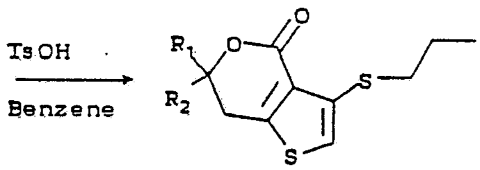


13

13 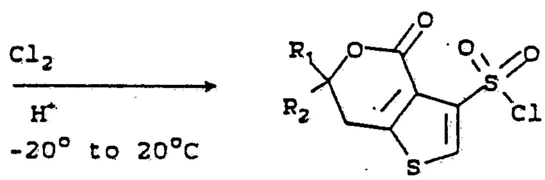


14

Equation 6a

13 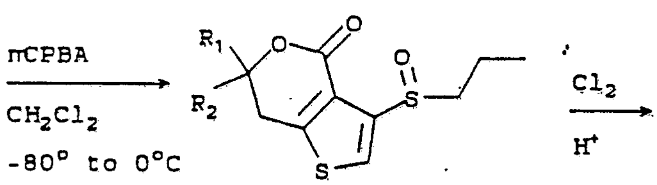

14

15

The sulfonyl chloride 14 may be transformed to the sulfonamide 2 by treatment with anhydrous ammonia in THF, ether or methylene chloride solution at temperatures ranging from -40° to 20°C, or with ammonium hydroxide in aqueous solution at 0° to 20°C, as shown in Equation 7.

Equation 7

14

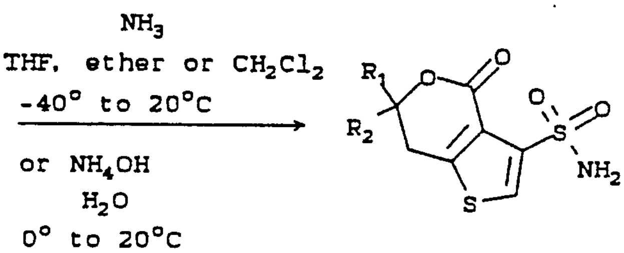


2

Agriculturally suitable salts of compounds of Formula I are also useful herbicides and can be prepared in a number of ways known to the art. For example, metal salts can be made by contacting compounds of Formula I with a solution of an alkali or alkaline earth metal salt having a sufficiently basic anion (e.g., hydroxide, alkoxide, or carbonate). Quaternary amine salts can be made by similar techniques.

Salts of compounds of Formula I can also be prepared by exchange of one cation for another. Cationic exchange can be effected by direct contact of an aqueous solution of a salt of a compound of Formula I (e.g., alkali or quaternary amine salt) with a solution containing the cation to be exchanged. This method is most effective when the desired salt containing the exchanged cation is insoluble in water and can be separated by filtration.

Exchange can also be effected by passing an aqueous solution of a salt of a compound of Formula I (e.g., an alkali metal or quaternary amine salt) through a column packed with a cation exchange resin containing the cation to be exchanged for that of the original salt and the desired product is eluted from the column. This method is particularly useful when the desired salt is water-soluble, e.g., a potassium, sodium or calcium salt.

Acid addition salts, useful in this invention, can be obtained by reacting a compound of Formula I with a suitable acid, e.g., p-toluenesulfonic acid, trichloroacetic acid or the like.

The preparation of the compounds of this invention is further illustrated by the following specific examples.

## Example 1

3-Bromo-4-(propylthio)thiophene

A solution of n-butyllithium (172 mL, 2.53 M, 0.434 mol) in ether (300 mL) was cooled to -78° C, and a solution of 3,4-dibromothiophene (100 g, 0.413 mol) in ether (300 mL) was dripped in at a rate sufficient to keep the temperature below -70° C. After stirring at -78° C for 15 minutes more, a solution of propyl disulfide (93 mL, 0.60 mol) in ether (120 mL) was added dropwise, forming a white suspension and causing an exotherm to -60° C. The mixture was allowed to warm to room temperature, poured over ice, acidified with HCl and extracted with ethyl acetate. The ethyl acetate layer was washed with brine, dried ($MgSO_4$) and concentrated, and the product was isolated by distillation. B.P. 95 - 98° C at 0.25 torr (33 Pa); yield 81 g; NMR ($CDCl_3$) δ: 1.02 (t, 3H), 1.6-1.8 (m, 2H), 2.84 (t, 2H), 7.10 and 7.31 (AB q, 2H).

## Example 2

3-Carboxy-4-(propylthio)thiophene

A solution of n-butyllithium (353 mL, 2.24 M, 0.79 mol) in ether (1 L) was cooled to -78° C and treated dropwise with a solution of 3-bromo-4-(propylthio)thiophene (178.8 g, 0.752 mol) at a rate sufficient to keep the temperature below -70° C. After stirring the amber solution at -78° C for another 15 minutes, solid carbon dioxide (99 g, 2.2 mol) was added in one portion, thereby causing a vigorous exotherm to -50° C and precipitation of a white solid. The mixture was warmed to 20° C and poured over ice, and then aqueous sodium hydroxide (1N) was added. The aqueous solution was washed with ether and then acidified with conc. HCl. The product was collected by filtration, washed with water and dried under high vacuum. M.P. 80-84° C; yield 129.5 g; NMR ($CDCl_3$) δ: 1.08 (t, 3H), 1.6-1.8 (m, 2H) 2.90 (t, 2H), 6.0-6.7 (broad hump 1H), 6.84 and 8.33 (AB q, 2H).

## Example 3

3-Carboxy-2-(2-hydroxyethyl)-4-(propylthio)thiophene

A solution of 3-carboxy-4-(propylthio)thiophene (8.0 g, 0.0396 mol) in THF (150 mL) was cooled to -30° C and treated dropwise with n-butyllithium (33 mL, 2.53 M, 0.0832 mol). After stirring at 20° C for 2 hours, the solution was cooled to -78° C and ethylene oxide (20 mL, 0.396 mol) was added dropwise. The mixture was warmed to 20° C, poured over ice, acidified and extracted with ethyl acetate. The organic phase was washed

with brine, dried and concentrated to give a tacky solid sufficiently pure to be used directly in the next reaction; yield 6.8 g.

Example 4

6,7-Dihydro-4-oxo-3-thiopropyl-4H-thieno[3,2-c]pyran

The product of Example 3 was dissolved in benzene (50 mL) and 0.5 g p-toluenesulfonic acid was added. The solution was warmed to reflux and water (0.7 mL) was removed with the aid of a Dean-Stark trap over 18 hours. The mixture was cooled, diluted with ethyl acetate and washed with aqueous sodium hydroxide (1N), water and brine, dried ($MgSO_4$) and concentrated to a tan solid. The solid was washed with a mixture of hexane and butyl chloride to give the product. M.P. 64-71°C; yield 4.74 g; NMR ($CDCl_3$) δ: 1.07 (t, 3H), 1.6-1.9 (m, 2H), 2.94 (t, 2H), 3.14 (t, 2H), 4.56 (t, 2H), 6.59 (s, 1H).

Example 5

6,7-Dihydro-4-oxo-4H-thieno[3,2-c]pyran-3-sulfonamide

The product of Example 4 (3.10 g, 0.136 mol) dissolved in a mixture of acetic acid (25 mL), propionic acid (10 mL) and water (0.8 mL), was cooled to 0°C and chlorine was added dropwise at a rate to maintain the temperature below 10°C. The solution was allowed to warm to 20°C and was stirred for 3 hours more. It was then poured over ice and extracted with methylene chloride, and the organic layer was washed with aqueous sodium bicarbonate and water, and dried ($MgSO_4$). The solvent was removed, and the solid residue was dissolved in THF (100 mL) and cooled to -50°C, and then ammonia was added dropwise. The mixture was warmed to 20°C, stirred overnight, diluted with ethyle acetate, washed with water and brine, dried ($MgSO_4$) and concentrated to give the product as a tan solid. M.P. 199-204°C; yield 1.96 g; NMR ($CDCl_3$) δ: 3.02 (t, J=6 Hz, 2H), 4.45 (t, J=6 Hz, 2H), 6.57 (br s, 2H), 7.74 (s, 1H).

Example 6

N-[[[4-(dimethylamino)-6-ethoxy-1,3,5-triazin-2-yl]amino]carbonyl]-6,7-dihydro-4-oxo-4H-thieno[3,2-c]pyran-3-sulfonamide

The product from Example 5 (0.15 g, 0.643 mmol) was dissolved in acetonitrile (4 mL), and phenyl N-(4-dimethylamino-6-ethoxy-1,3,5-triazin-2-yl)carbamate (0.25 g, 0.836 mmol) and DBU (125 µL, 0.836 mmol) were added. After the solution was stirred for 1 hour, it was diluted with water (10 mL) and acidified with hydrochloric acid (1N). The product was isolated by filtration, washed with water, butyl chloride and ether, and dried to yield 0.17 g of solid. M.P. 189-194°C; IR (nujol) $cm^{-1}$: 1725, 1710, 1600, 1535; NMR ($CDCl_3$) δ: 1.38 (t, 3H), 3.1-3.3 (m, 8H), 4.41 (q, 2H)(, 4.59 (t, 2H), 7.22 (br s, 1H), 8.30 (s, 1H), 13.18 (br s, 1H).

Following the procedures described earlier and exemplified in Examples 1-6, one skilled in the art can prepare the compounds of Table I.

TABLE 1

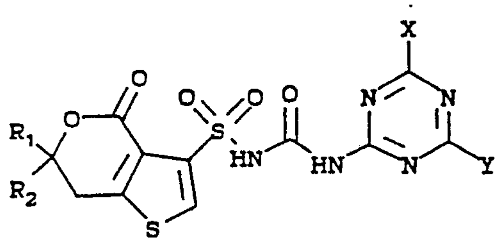

| $R_1$ | $R_2$ | X | Y |
|---|---|---|---|
| H | H | $OCH_3$ | $NHCH_3$ |
| H | H | $OCH_3$ | $N(CH_3)_2$ |
| H | H | $OCH_3$ | $N(CH_3)OCH_3$ |
| H | H | $OCH_3$ | $OCH_2CH_3$ |
| H | H | $OCH_3$ | $OCH_3$ |
| H | H | $OCH_3$ | $OCH_2CF_3$ |
| H | H | $OCH_3$ | $CH_2CH_3$ |
| H | H | $OCH_3$ | $CH(CH_3)_2$ |
| H | H | $OCH_3$ | cyclopropyl |
| H | H | $OCH_2CH_3$ | $NHCH_3$ |
| H | H | $OCH_2CH_3$ | $N(CH_3)_2$ |
| H | H | $OCH_2CH_3$ | $N(CH_3)OCH_3$ |
| H | H | $OCH_2CH_3$ | $OCH_2CH_3$ |
| H | H | $OCH_2CH_3$ | $OCH_3$ |
| H | H | $OCH_2CH_3$ | $OCH_2CF_3$ |
| H | H | $OCH_2CH_3$ | $CH_2CH_3$ |
| H | H | $OCH_2CH_3$ | $CH(CH_3)_2$ |
| H | H | $OCH_2CH_3$ | cyclopropyl |

| $R_1$ | $R_2$ | X | Y |
|---|---|---|---|
| $CH_3$ | H | $OCH_3$ | $NHCH_3$ |
| $CH_3$ | H | $OCH_3$ | $N(CH_3)_2$ |
| $CH_3$ | H | $OCH_3$ | $N(CH_3)OCH_3$ |
| $CH_3$ | H | $OCH_3$ | $OCH_2CH_3$ |
| $CH_3$ | H | $OCH_3$ | $OCH_3$ |
| $CH_3$ | H | $OCH_3$ | $OCH_2CF_3$ |
| $CH_3$ | H | $OCH_3$ | $CH_2CH_3$ |
| $CH_3$ | H | $OCH_3$ | $CH(CH_3)_2$ |
| $CH_3$ | H | $OCH_3$ | cyclopropyl |
| $CH_3$ | H | $OCH_2CH_3$ | $NHCH_3$ |
| $CH_3$ | H | $OCH_2CH_3$ | $N(CH_3)_2$ |
| $CH_3$ | H | $OCH_2CH_3$ | $N(CH_3)OCH_3$ |
| $CH_3$ | H | $OCH_2CH_3$ | $OCH_2CH_3$ |
| $CH_3$ | H | $OCH_2CH_3$ | $OCH_3$ |
| $CH_3$ | H | $OCH_2CH_3$ | $OCH_2CF_3$ |
| $CH_3$ | H | $OCH_2CH_3$ | $CH_2CH_3$ |
| $CH_3$ | H | $OCH_2CH_3$ | $CH(CH_3)_2$ |
| $CH_3$ | H | $OCH_2CH_3$ | cyclopropyl |
| $CH_3$ | $CH_3$ | $OCH_3$ | $NHCH_3$ |
| $CH_3$ | $CH_3$ | $OCH_3$ | $N(CH_3)_2$ |
| $CH_3$ | $CH_3$ | $OCH_3$ | $N(CH_3)OCH_3$ |
| $CH_3$ | $CH_3$ | $OCH_3$ | $OCH_2CH_3$ |
| $CH_3$ | $CH_3$ | $OCH_3$ | $OCH_3$ |
| $CH_3$ | $CH_3$ | $OCH_3$ | $OCH_2CF_3$ |
| $CH_3$ | $CH_3$ | $OCH_3$ | $CH_2CH_3$ |
| $CH_3$ | $CH_3$ | $OCH_3$ | $CH(CH_3)_2$ |
| $CH_3$ | $CH_3$ | $OCH_3$ | cyclopropyl |
| $CH_3$ | $CH_3$ | $OCH_2CH_3$ | $NHCH_3$ |
| $CH_3$ | $CH_3$ | $OCH_2CH_3$ | $N(CH_3)_2$ |
| $CH_3$ | $CH_3$ | $OCH_2CH_3$ | $N(CH_3)OCH_3$ |

| $R_1$ | $R_2$ | X | Y |
|---|---|---|---|
| $CH_3$ | $CH_3$ | $OCH_2CH_3$ | $OCH_2CH_3$ |
| $CH_3$ | $CH_3$ | $OCH_2CH_3$ | $OCH_3$ |
| $CH_3$ | $CH_3$ | $OCH_2CH_3$ | $OCH_2CF_3$ |
| $CH_3$ | $CH_3$ | $OCH_2CH_3$ | $CH_2CH_3$ |
| $CH_3$ | $CH_3$ | $OCH_2CH_3$ | $CH(CH_3)_2$ |
| $CH_3$ | $CH_3$ | $OCH_2CH_3$ | cyclopropyl |
| $CH_2CH_3$ | H | $OCH_3$ | $NHCH_3$ |
| $CH_2CH_3$ | H | $OCH_3$ | $N(CH_3)_2$ |
| $CH_2CH_3$ | H | $OCH_3$ | $N(CH_3)OCH_3$ |
| $CH_2CH_3$ | H | $OCH_3$ | $OCH_2CH_3$ |
| $CH_2CH_3$ | H | $OCH_3$ | $OCH_3$ |
| $CH_2CH_3$ | H | $OCH_3$ | $OCH_2CF_3$ |
| $CH_2CH_3$ | H | $OCH_3$ | $CH_2CH_3$ |
| $CH_2CH_3$ | H | $OCH_3$ | $CH(CH_3)_2$ |
| $CH_2CH_3$ | H | $OCH_3$ | cyclopropyl |
| $CH_2CH_3$ | H | $OCH_2CH_3$ | $NHCH_3$ |
| $CH_2CH_3$ | H | $OCH_2CH_3$ | $N(CH_3)_2$ |
| $CH_2CH_3$ | H | $OCH_2CH_3$ | $N(CH_3)OCH_3$ |
| $CH_2CH_3$ | H | $OCH_2CH_3$ | $OCH_2CH_3$ |
| $CH_2CH_3$ | H | $OCH_2CH_3$ | $OCH_3$ |
| $CH_2CH_3$ | H | $OCH_2CH_3$ | $OCH_2CF_3$ |
| $CH_2CH_3$ | H | $OCH_2CH_3$ | $CH_2CH_3$ |
| $CH_2CH_3$ | H | $OCH_2CH_3$ | $CH(CH_3)_2$ |
| $CH_2CH_3$ | H | $OCH_2CH_3$ | cyclopropyl |

Formulations

Useful formulations of the compound of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

| | Weight Percent* Active Ingredient | Diluent(s) | Surfactant(s) |
|---|---|---|---|
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser one for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147ff. and "Perry's Chemical Engineer's Handbook", 5th Ed., McGraw-Hill, New York, 1973, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed Control as a Science", John Wiley and Sons, Inc., New York, 1961, pp. 81-96; and J. D. Fryer and S. A. Evans, "Weed Control Handbook", 5th Ed., Blackwell Scientific Publications, Oxford, 1968, pp. 101-103.

In the following Examples, all parts are by weight unless otherwise indicated.

Example 7

Wettable Powder

| | |
|---|---|
| N-[[[4-(dimethylamino)-6-ethoxy-1,3,5-triazin-2-yl]amino]carbonyl]-6,7-dihydro-4-oxo-4H-thieno[3,2-c]pyran-3-sulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example 8

Wettable Powder

| | |
|---|---|
| N-[[[4-(dimethylamino)-6-ethoxy-1,3,5-triazin-2-yl]amino]carbonyl]-6,7-dihydro-4-oxo-4H-thieno[3,2-c]pyran-3-sulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially below 10 microns in diameter. The product is reblended before packaging.

Example 9

Granule

| | |
|---|---|
| Wettable Powder of Example 8 | 5% |
| attapulgite granules (U.S.S. 20 to 40 mesh; 0.84-0.42 mm) | 95% |

A slurry of wettable powder containing 25% solids is sprayed on the surface of the attapulgite granules in a double-cone blender. The granules are dried and packaged.

Example 10

Extruded Pellet

| | |
|---|---|
| N-[[[4-(dimethylamino)-6-ethoxy-1,3,5-triazin-2-yl]amino]carbonyl]-6,7-dihydro-4-oxo-4H-thieno[3,2-c]pyran-3-sulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 11

Low Strength Granule

| | |
|---|---|
| N-[[[4-(dimethylamino)-6-ethoxy-1,3,5-triazin-2-yl]amino]carbonyl]-6,7-dihydro-4-oxo-4H-thieno[3,2-c]pyran-3-sulfonamide | 0.1% |
| attapulgite granules (U.S.S. 20 to 40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 12

Granule

| | |
|---|---|
| N-[[[4-(dimethylamino)-6-ethoxy-1,3,5-triazin-2-yl]amino]carbonyl]-6,7-dihydro-4-oxo-4H-thieno[3,2-c]pyran-3-sulfonamide | 80% |
| wetting agent | 1% |
| crude ligninsulfonate salt (containing 5 to 20% of the natural sugars) | 10% |
| attapulgite clay | 9% |

The ingredients are blended and milled to pass through a 100 mesh screen. This material is then added to a

fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14 to 100 mesh (1410 to 149 microns), and packaged for use.

Example 13

Low Strength Granule

| | |
|---|---|
| N-[[[4-(dimethylamino)-6-ethoxy-1,3,5-triazin-2-yl]amino-]carbonyl]-6,7-dihydro-4-oxo-4H-thieno[3,2-c]pyran-3-sulfonamide | 1% |
| N,N-dimethylformamide | 9% |
| attapulgite granules (U.S.S 20 to 40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 14

Aqueous Suspension

| | |
|---|---|
| N-[[[4-(dimethylamino)-6-ethoxy-1,3,5-triazin-2-yl]amino-]carbonyl]-6,7-dihydro-4-oxo-4H-thieno[3,2-c]pyran-3-sulfonamide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 15

Solution

| | |
|---|---|
| N-[[[4-(dimethylamino)-6-ethoxy-1,3,5-triazin-2-yl]amino-]carbonyl]-6,7-dihydro-4-oxo-4H-thieno[3,2-c]pyran-3-sulfonamide, sodium salt | 5% |
| water | 95% |

The salt is added directly to the water with stirring to produce the solution, which may then be packaged for use.

Example 16

High Strength Concentrate

| | |
|---|---|
| N-[[[4-(dimethylamino)-6-ethoxy-1,3,5-triazin-2-yl]amino-]carbonyl]-6,7-dihydro-4-oxo-4H-thieno[3,2-c]pyran-3-sulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 17

Wettable Powder

| | |
|---|---|
| N-[[[4-(dimethylamino)-6-ethoxy-1,3,5-triazin-2-yl]amino-]carbonyl]-6,7-dihydro-4-oxo-4H-thieno[3,2-c]pyran-3-sulfonamide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 18

Wettable Powder

| | |
|---|---|
| N-[[[4-(dimethylamino)-6-ethoxy-1,3,5-triazin-2-yl]amino-]carbonyl]-6,7-dihydro-4-oxo-4H-thieno[3,2-c]pyran-3-sulfonamide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 19

Oil Suspension

| | |
|---|---|
| N-[[[4-(dimethylamino)-6-ethoxy-1,3,5-triazin-2-yl]amino-]carbonyl]-6,7-dihydro-4-oxo-4H-thieno[3,2-c]pyran-3-sulfonamide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 20

Dust

| | |
|---|---|
| N-[[[4-(dimethylamino)-6-ethoxy-1,3,5-triazin-2-yl]amino-]carbonyl]-6,7-dihydro-4-oxo-4H-thieno[3,2-c]pyran-3-sulfonamide | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Example 21

Oil Suspension

| | |
|---|---|
| N-[[[4-(dimethylamino)-6-ethoxy-1,3,5-triazin-2-yl]amino-]carbonyl]-6,7-dihydro-4-oxo-4H-thieno[3,2-c]pyran-3-sulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 22

Wettable Powder

| | |
|---|---|
| N-[[[4-(dimethylamino)-6-ethoxy-1,3,5-triazin-2-yl]amino-]carbonyl]-6,7-dihydro-4-oxo-4H-thieno[3,2-c]pyran-3-sulfonamide | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

Utility

Test results indicate that compounds of the present invention are highly active pre-emergent or post-emergent herbicides or plant growth regulants. Many of them have utility for broad-spectrum pre-and/or post-emergence weed control in areas where control of vegetation is desired, such as around storage tanks, parking lots, drive-in theaters, billboards, highways, railroad structures, and fallow. A substantial number of the compounds are particularly useful for selective weed control in beets, cole crops, flax (Linum usitatissimum), lentil (Lens culinaris), and safflower (Carthamus tinctorius). Beets are those crops of Beta spp. that include, but are not limited to, fodder beets, sugar beets, and table beets. Cole crops are crops of Brassica spp. which include but are not limited to, broccoli, brussel sprouts, cabbage, cauliflower, kale, kohlrabi, pak choi, rape, and turnips. Alternatively, the subject compounds are useful to modify plant growth and as citrus abscission harvest aids.

Rates of application for compounds of this invention are determined by a number of factors, including their use as plant growth modifiers or as herbicides, crop species involved, types of weeds to be controlled, weather and climate, formulations selected, mode of application, amount of foliage present, etc. In general terms, the subject compounds should be applied at levels of around 0.001 to 20 kg/ha, the lower rates being suggested for use on lighter soils and/or those having a low organic matter content, for plant growth modification, or for situations where only short term persistence is required, such as herbicide for fallow land. Preferred rates of application are from approximately 0.004 to approximately 0.5 kg/ha. One skilled in the art can easily determine application rates necessary for the desired level of weed control.

Compounds of this invention may be used in combination with other commercial herbicides, insecticides, or fungicides. The following list exemplifies some of the herbicides suitable for use in mixtures.

| Common Name | Chemical Name |
|---|---|
| alachlor | 2-chloro-N-(2,6-diethylphenyl)-N-(methoxymethyl)acetamide |
| alloxydim | 2-[1-(N-allyloxyamino)butylidene]-4-methoxycarbonyl-5,5-dimethylcyclohexane-1,3-dione |
| amitrole | 1H-1,2,4-triazol-3-amine |
| asulam | methyl [(4-aminophenyl)sulfonyl]carbamate |
| atrazine | 6-chloro-N-ethyl-N'-(1-methylethyl)-1,3,5-triazine-2,4-diamine |
| barban | 4-chloro-2-butynyl 3-chlorocarbamate |
| BAS-518 | 7-chloro-3-methylquinoline-8-carboxylic acid |
| benazolin | 4-chloro-2-oxobenzothiazolin-3-yl acetic acid |
| bentazon | 3-(1-methylethyl)-(1H)-2,1,3-benzothiadiazin-4(3H)-one, 2,2-dioxide |
| benzoylprop | N-benzoyl-N-(3,4-dichlorophenyl)-DL-alanine |
| bromoxynil | 3,5-dibromo-4-hydroxybenzonitrile |
| butylate | S-ethyl bis(2-methylpropyl)carbamothioate |
| carbetamide | D-N-ethyl-2-(phenylcarbamoyloxy)propioamide |
| CDAA | 2-chloro-N,N-di-2-propenylacetamide |
| chlorpropham | 1-methylethyl 3-chlorophenylcarbamate |
| clopyralid | 3,6-dichloro-2-pyridinecarboxylic acid |
| cyanazine | 2-[[4-chloro-6-(ethylamino)-1,3,5-triazin-2-yl]amino]-2-methylpropanenitrile |
| cycloate | S-ethyl cyclohexylethylcarbamothioate |
| dalapon | 2,2-dichloropropanoic acid |

| Common Name | Chemical Name |
|---|---|
| desmediphan | ethyl [3-[[(phenylamino)carbonyl]oxy]-phenyl]carbamate |
| diallate | S-(2,3-dichloro-2-propenyl)bis(1-methylethyl)carbamothioate |
| dicamba | 3,6-dichloro-2-methoxybenzoic acid |
| dichlofop | (±)-2-[4-(2,4-dichlorophenoxy)phenoxy]-propanoic acid, methyl ester |
| diethatyl | N-(chloroacetyl)-N-(2,6-diethylphenyl)-glycine |
| difenzoquat | 1,2-dimethyl-3,5-diphenyl-1H-pyrazolium |
| dimefuron | 4-[2-chloro-4-(3,3-dimethylureido)-phenyl]-2-t-butyl-1,3,4-oxadiazolin-5-one |
| diquat | 6,7-dihydrodipyrido[1,2-a:2',1'-c]-pyrazinedium ion |
| DPX-A7881 | 2-[[(4-ethoxy-6-methylamino-1,3,5-triazin-2-yl)aminocarbonyl]amino-sulfonyl]benzoic acid, methyl ester |
| endothall | 7-oxabicyclo[2.2.1]heptane-2,3-dicarboxylic acid |
| EPTC | S-ethyl dipropylcarbamothioate |
| ethalfluralin | N-ethyl-N-(2-methyl-2-propenyl)-2,6-dinitro-4-(trifluoromethyl)-benzenamine |
| ethofumesate | (±)-2-ethoxy-2,3-dihydro-3,3-dimethyl-5-benzofuranyl methanesulfonate |
| fluazifop | (±)-2-[4-[[5-(trifluoromethyl)-2-pyridinyl]oxy]phenoxy]propanoic acid |
| fluazifop-P | (R)-2-[4-[[5-(trifluoromethyl)-2-pyridinyl]oxy]phenoxy]propanoic acid |

| Common Name | Chemical Name |
|---|---|
| glufosinate | DL-homoalanin-4-yl(methyl)phosphinic acid |
| glyphosate | N-(phosphonomethyl)glycine |
| lenacil | 3-cyclohexyl-6,7-dihydro-1H-cyclopenta-pyrimidine-2,4(3H,5H)-dione |
| MCPA | (4-chloro-2-methylphenoxy)acetic acid |
| metamitron | 4-amino-3-methyl-6-phenyl-1,2,4-triazin-5(4H)-one |
| metazachlor | α-chloro-N-(1-pyrazolylmethyl)aceto-2',6'-xylidide |
| metribuzin | 4-amino-6-(1,1-dimethylethyl)-3-(methyl-thio)-1,2,4-triazin-5(4H)-one |
| napropamide | N,N-diethyl-2-(1-naphthalenyloxy)-propanamide |
| paraquat | 1,1'-dimethyl-4,4'-dipyridinium ion |
| pebulate | S-propyl butylethylcarbamothioate |
| phenmedipham | 3-[(methoxycarbonyl)amino]phenyl (3-methylphenyl)carbamate |
| picloram | 4-amino-3,5,6-trichloro-2-pyridine-carboxylic acid |
| pronamide | 3,5-dichloro-N-(1,1-dimethyl-2-propyn-yl)benzamide |
| propachlor | 2-chloro-N-(1-methylethyl)-N-phenylacetamide |
| propham | 1-methylethyl phenylcarbamate |
| pyrazon | 5-amino-4-chloro-2-phenyl-3(2H)-pyridazinone |
| pyridate | octyl-O-(6-chloro-3-phenylpyridazin-4-yl)carbonothioate |

| Common Name | Chemical Name |
|---|---|
| quizalofop ethyl | (±)-2-[4-[(6-chloro-2-quinoxalinyl)-oxy]phenoxy]propanoic acid, ethyl ester |
| sethoxydim | 2-[1-(ethoxyimino)butyl]-5-[2-(ethyl-thio)propyl]-3-hydroxy-2-cyclohexen-1-one |
| TCA | trichloroacetic acid |
| tebutam | N-benzyl-N-isopropylpivalamide |
| triallate | S-(2,3,3-trichloro-2-propenyl) bis(1-methylethyl)carbamothioate |
| trifluralin | 2,6-dinitro-N,N-dipropyl-4-(tri-fluoromethyl)benzenamine |
| 2,4-D | (2,4-dichlorophenoxy)acetic acid |

Herbicidal properties of the subject compounds were discovered in a number of greenhouse tests. Test procedures and results follow.

COMPOUNDS

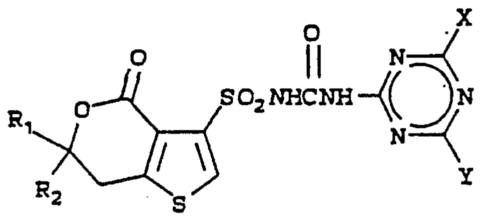

| CMPD | $R_1$ | $R_2$ | X | Y | m.p.(°C) |
|---|---|---|---|---|---|
| 1 | H | H | $OCH_2CH_3$ | $N(CH_3)_2$ | 189-194 |
| 2 | H | H | $OCH_2CH_3$ | $NHCH_3$ | 225-232 |
| 3 | H | H | $OCH_2CH_3$ | $OCH_2CH_3$ | 169-173 |
| 4 | $CH_3$ | H | $OCH_2CH_3$ | $NHCH_3$ | 164-166 |
| 5 | $CH_3$ | H | $OCH_2CH_3$ | $N(CH_3)_2$ | 221-223 |
| 6 | H | H | $OCH_2CH_3$ | cyclopropyl | 193-195 |
| 7 | H | H | $OCH_2CH_3$ | $CH(CH_3)_2$ | 170-172 |
| 8 | $CH_3$ | $CH_3$ | $OCH_2CH_3$ | $NHCH_3$ | 172-187 |
| 9 | $CH_3$ | $CH_3$ | $OCH_2CH_3$ | $N(CH_3)_2$ | 229-232 |
| 10 | H | H | $OCH_2CH_3$ | $CH_2CH_3$ | 168-170 |
| 11 | $CH_3$ | H | $OCH_3$ | $OCH_3$ | 207-212 |
| 12 | $CH_3$ | $CH_3$ | $OCH_3$ | $OCH_3$ | 218-220 |
| 13 | $CH_2CH_3$ | H | $OCH_3$ | $OCH_3$ | 226-228 |
| 14 | $CH_2CH_3$ | H | $OCH_2CH_3$ | $NHCH_3$ | 176-186 |
| 15 | $CH_2CH_3$ | H | $OCH_2CH_3$ | $N(CH_3)_2$ | 222-225 |

TEST A

Seeds of barley (Hordeum vulgare), barnyardgrass (Echinochloa crus-galli), cheatgrass (Bromus secalinus) cocklebur (Xanthium pensylvanicum), corn (Zea mays), cotton (Gossypium hirsutum), crabgrass (Digitaria spp.), giant foxtail (Setaria faberi), morningglory (Impomoea spp.), rice (Oryza sativa), sorghum (Sorghum bicolor), soybean (Glycine max), sugar beet (Beta vulgaris), velvetleaf (Abutilon theophrasti), wheat (Triticum aestivum), and wild oat (Avena fatua) and purple nutsedge (Cyperus rotundus) tubers were planted and treated preemergence with test chemicals dissolved in a non-phytotoxic solvent. At the same time, these crop and weed species were also treated with postemergence applications of test chemicals. Plants ranged in height from two to eighteen cm (two to three leaf stage) for postemergence treatments. Treated plants and controls were maintained in a greenhouse for sixteen days, after which all species were compared to controls and visually evaluated. The ratings, summarized in Table A, are based on a scale of 0 to 10 where 0 is no effect and 10 is complete control. A dash (-) response means no test result. The accompanying descriptive symbols

have the following meanings:
C = chlorosis/necrosis;
E = inhibition of emergence;
G = growth retardation;
H = formative effect; and
U = unusual pigmentation.

Table A

| | Cmpd 1 | | Cmpd 2 | |
|---|---|---|---|---|
| RATE (g/ha) | 50 | 10 | 50 | 10 |
| POSTEMERGENCE | | | | |
| Barley | 9C | 9G | 5G | 5G |
| Barnyardgrass | 3C,9H | 3G | 0 | 0 |
| Cheatgrass | 9G | 9G | 8G | 5G |
| Cocklebur | 4C,9G | 5G | 5G | 0 |
| Corn | 3C,9G | 9G | 4C,9G | 9G |
| Cotton | 3C,8H | 5G | 3C,6G | 0 |
| Crabgrass | 5C,9G | 3C,8G | 2C,5G | 4G |
| Giant foxtail | 9C | 9G | 4C,9G | 5G |
| Morningglory | 5C,9G | 2C,7G | 4C,8H | 2G |
| Nutsedge | 2G | 0 | 3G | 0 |
| Rice | 9C | 4C,9G | 9G | 7G |
| Sorghum | 4C,9G | 3C,9G | 2G | 3C,7G |
| Soybean | 5C,9H | 2C,5H | 4C,8G | 3C,7H |
| Sugar beet | 2C,4G | 2G | 0 | 0 |
| Velvetleaf | 9C | 4C,9G | 3C,7H | 2G |
| Wheat | 9G | 8G | 8G | 7G |
| Wild oat | 5C,9G | 9G | 8G | 8G |
| PREEMERGENCE | | | | |
| Barley | 4G | 0 | 2G | 0 |
| Barnyardgrass | 2G | 0 | 0 | 0 |
| Cheatgrass | 7G | 0 | 5G | 0 |
| Cocklebur | 0 | 0 | 0 | 0 |
| Corn | 2C,8G | 0 | 3C,7G | 0 |
| Cotton | 1C,2G | 0 | 0 | 0 |
| Crabgrass | 4C,9G | 4G | 3C,8G | 0 |
| Giant foxtail | 6G | 2G | 7G | 0 |
| Morningglory | 2C,3G | 0 | 0 | 0 |
| Nutsedge | 0 | 0 | 0 | 0 |
| Rice | 9H | 0 | 6G | 0 |
| Sorghum | 3C,8H | 0 | 9H | 2C |
| Soybean | 2C,4G | 0 | 0 | 0 |
| Sugar beet | 4G | 0 | 0 | 0 |
| Velvetleaf | 2C,6G | 0 | 0 | 0 |
| Wheat | 7G | 0 | 7H | 0 |
| Wild oat | 2C,5G | 0 | 4G | 0 |

Table A

| | Cmpd 3 | | Cmpd 4 | |
|---|---|---|---|---|
| RATE (g/ha) | 50 | 10 | 50 | 10 |
| POSTEMERGENCE | | | | |
| Barley | 2C,8G | 2C,4G | 3C,9G | 7G |
| Barnyardgrass | 4C,8G | 3C,5H | 3C,7H | 3C,5H |
| Cheatgrass | 5C,9G | 3C,6G | 8G | 6G |
| Cocklebur | 9C | 4C,9H | 2C,6G | 2C,2H |
| Corn | 5C,9G | 2C,9H | 4C,9G | 3C,9G |
| Cotton | 3C,6G | 2G | 3C,6G | 2C,4H |
| Crabgrass | 4C,8G | 3C,5G | 3C,7G | 2G |
| Giant foxtail | 4C,8H | 3C,7G | 4C,9G | 3C,8G |
| Morningglory | 5C,9G | 4C,9G | 4C,8H | 3C,7H |
| Nutsedge | 4G | 5G | 3G | - |
| Rice | 9C | 5C,9G | 5C,9G | 5C,9G |
| Sorghum | 4C,9G | 3C,9G | 4C,9G | 3C,9G |
| Soybean | 4C,9G | 3C,7G | 4C,9H | 3C,7G |
| Sugar beet | 5C,9G | 0 | 2H | 0 |
| Velvetleaf | 5C,9G | 3C,7H | 3C,7H | 0 |
| Wheat | 9G | 7G | 9G | 9G |
| Wild oat | 3C,9G | 3C,6G | 3C,9G | 2C,8G |
| PREEMERGENCE | | | | |
| Barley | 2C | 0 | 0 | 0 |
| Barnyardgrass | 3C,7G | 0 | 3G | 0 |
| Cheatgrass | 0 | 0 | 5G | 0 |
| Cocklebur | 0 | 0 | 3G | 2G |
| Corn | 2C,4G | 0 | 3C,8G | 0 |
| Cotton | 5G | 0 | 2C,6G | 0 |
| Crabgrass | 2G | 0 | 6G | 6G |
| Giant foxtail | 3C,8G | 0 | 2C,6G | 3G |
| Morningglory | 3G | 0 | 0 | 0 |
| Nutsedge | 0 | 0 | 0 | 0 |
| Rice | 2C,7G | 0 | 3C,7G | 0 |
| Sorghum | 2C,5G | 2C | 3C,7G | 0 |
| Soybean | 2H | 0 | 3C,6H | 2G |
| Sugar beet | 5H | 0 | 0 | 0 |
| Velvetleaf | 3C,3H | 0 | 2H | 0 |
| Wheat | 3C,8G | 0 | 6G | 0 |
| Wild oat | 2C,6G | 0 | 3C,6G | 0 |

Table A

| | Cmpd 5 | | Cmpd 6 | |
|---|---|---|---|---|
| RATE (g/ha) | 50 | 10 | 50 | 10 |
| POSTEMERGENCE | | | | |
| Barley | 4C,8G | 9G | 2C,6G | 2G |
| Barnyardgrass | 3C,8G | 5G | 4C,8H | 3C,5G |
| Cheatgrass | 8G | 7G | 3C,7G | 2C,3G |
| Cocklebur | 4C,9G | 8G | 9C | 3C,9G |
| Corn | 5C,9G | 9G | 3C,9G | 9G |
| Cotton | 3C,8G | 3G | 3C,8H | 3C,7G |
| Crabgrass | 3C,8G | 8G | 3C,7G | 7G |
| Giant foxtail | 5C,9G | 3C,8G | 4C,8G | 3C,7G |
| Morningglory | 5C,9G | 4C,9G | 4C,9G | 3C,8H |
| Nutsedge | 3C,7G | 3G | 5G | - |
| Rice | 4C,9G | 4C,9G | 5C,9G | 4C,9G |
| Sorghum | 9G | 9G | 2C,9G | 4C,9G |
| Soybean | 3C,9G | 3C,7H | 3C,8H | 2C,2H |
| Sugar beet | 3C,3G | 3G | 3C,4H | 0 |
| Velvetleaf | 3C,8H | 7G | 4C,8H | 3C,8G |
| Wheat | 9G | 5C,9G | 8G | 7G |
| Wild oat | 9G | 2C,6G | 3C,6G | 2C |
| PREEMERGENCE | | | | |
| Barley | 6G | 0 | 7G | 0 |
| Barnyardgrass | 0 | 0 | 5G | 0 |
| Cheatgrass | 7G | 0 | 5G | 0 |
| Cocklebur | 0 | 0 | 0 | 0 |
| Corn | 3C,9G | 2C,4G | 3C,7G | 2G |
| Cotton | 0 | 0 | 2C,6G | - |
| Crabgrass | 3C,9H | 2G | 3C,8G | 0 |
| Giant foxtail | 4C,9H | 2G | 0 | 0 |
| Morningglory | 3G | 0 | 3C,7H | 0 |
| Nutsedge | 0 | 0 | 0 | 0 |
| Rice | 2C,6H | 0 | 4C,8G | 2G |
| Sorghum | 3C,7H | 2C,4H | 4C,8G | 2C,2G |
| Soybean | 2C,2G | 0 | 2C,7G | 2C,1H |
| Sugar beet | 0 | 0 | 3C,5G | 3H |
| Velvetleaf | 2H | 0 | 4H | 0 |
| Wheat | 3C,8H | 0 | 7G | 0 |
| Wild oat | 4G | 2G | 3C,5G | 2C |

Table A

| | Cmpd 7 | | Cmpd 8 | |
|---|---|---|---|---|
| RATE (g/ha) | 50 | 10 | 50 | 10 |
| POSTEMERGENCE | | | | |
| Barley | 5G | 0 | 3C,5G | 2G |
| Barnyardgrass | 2C,7G | 2G | 2H | 0 |
| Cheatgrass | 0 | 0 | 2C,7G | 0 |
| Cocklebur | 9C | 2C,7G | 0 | 0 |
| Corn | 2C,9G | 3C,8H | 3C,9H | 2C,7G |
| Cotton | 7G | 2H | 0 | 0 |
| Crabgrass | 0 | 0 | 3G | 0 |
| Giant foxtail | 3G | 2G | 3C,8G | 2G |
| Morningglory | 2C,8G | 2C,7G | 2H | 1H |
| Nutsedge | 0 | 0 | 0 | 0 |
| Rice | 4C,9G | 2C,8G | 4C,9G | 2C,8G |
| Sorghum | 3C,9G | 2C,8G | 2C,3G | 0 |
| Soybean | 3C,7G | 3H | 3C,8H | 1H |
| Sugar beet | 4G | 0 | 0 | 0 |
| Velvetleaf | 7G | 6G | 4G | 0 |
| Wheat | 7G | 3G | 9G | 5G |
| Wild oat | 0 | 0 | 2C,7G | 0 |
| PREEMERGENCE | | | | |
| Barley | 7G | 3C,5G | 0 | 0 |
| Barnyardgrass | 1H | 0 | 0 | 0 |
| Cheatgrass | 2C,3G | 0 | 0 | 0 |
| Cocklebur | 2C | 0 | 4G | 0 |
| Corn | 2C,7G | 3C,5G | 2G | 0 |
| Cotton | 4G | 2G | 0 | 0 |
| Crabgrass | 3G | 0 | 0 | 0 |
| Giant foxtail | 3G | 0 | 0 | 0 |
| Morningglory | 7G | 2C,3G | 0 | 0 |
| Nutsedge | 0 | 0 | 0 | 0 |
| Rice | 4G | 3G | 2G | 0 |
| Sorghum | 3C,7G | 6G | 0 | 0 |
| Soybean | 3C,6G | 2C,2H | 0 | 0 |
| Sugar beet | 1H | 0 | 3G | 0 |
| Velvetleaf | 6H | 0 | 2G | 0 |
| Wheat | 3C,8G | 0 | 0 | 0 |
| Wild oat | 4G | 0 | 0 | 0 |

Table A

| | Cmpd 9 | | Cmpd 10 | |
|---|---|---|---|---|
| RATE (g/ha) | 50 | 10 | 50 | 10 |
| POSTEMERGENCE | | | | |
| Barley | 3C,9G | 3C,7G | 5C,9G | 3C,9G |
| Barnyardgrass | 3C,5G | 0 | 5C,9G | 3C,9H |
| Cheatgrass | 7G | 0 | 9C | 3C,8G |
| Cocklebur | 2C,8H | 2G | 10C | 9C |
| Corn | 3C,9G | 3C,7G | 4U,9C | 4C,9G |
| Cotton | 3C,3H | 2H | 10C | 4C,9G |
| Crabgrass | 5G | 0 | 3C,9G | 7G |
| Giant foxtail | 3C,8G | 3G | 9C | 4C,9G |
| Morningglory | 2C,5G | 2C,3G | 10C | 10C |
| Nutsedge | 0 | 0 | 9G | 7G |
| Rice | 2C,9G | 3C,6G | 6C,9G | 6C,9G |
| Sorghum | 3C,8H | 2C,2G | 5C,9G | 4C,9G |
| Soybean | 3C,8G | 3C,5H | 6C,9G | 5C,9G |
| Sugar beet | 0 | 0 | 9C | 4C,8H |
| Velvetleaf | 7G | 0 | 9C | 5C,9G |
| Wheat | 9G | 8G | 3C,9G | 2C,9G |
| Wild oat | 2C,6G | 4G | 2C,9G | 3G |
| PREEMERGENCE | | | | |
| Barley | 0 | 0 | 9H | 8G |
| Barnyardgrass | 0 | 0 | 3C,9H | 3C,8G |
| Cheatgrass | 0 | 0 | 3C,8H | 2C,6G |
| Cocklebur | 2G | 0 | 9H | 8G |
| Corn | 2G | 0 | 9G | 3C,9G |
| Cotton | 0 | 0 | 3C,7G | 6G |
| Crabgrass | 0 | 0 | 7G | 4G |
| Giant foxtail | 3G | 0 | 4C,9H | 3C,8G |
| Morningglory | 0 | 0 | 9G | 9G |
| Nutsedge | 0 | 0 | 3C,5G | 0 |
| Rice | 0 | 0 | 10E | 9H |
| Sorghum | 2C,2G | 0 | 4C,9G | 3C,8G |
| Soybean | 2G | 0 | 9H | 3C,7H |
| Sugar beet | 0 | 0 | 9G | 8G |
| Velvetleaf | 0 | 0 | 4C,9G | 4C,9G |
| Wheat | 0 | 0 | 9H | 9H |
| Wild oat | 0 | 0 | 3C,7G | 3C,6G |

Table A

| | Cmpd 11 | | Cmpd 12 | |
|---|---|---|---|---|
| RATE (g/ha) | 50 | 10 | 50 | 10 |
| POSTEMERGENCE | | | | |
| Barley | 3C,9G | 9G | 2C,5G | 3G |
| Barnyardgrass | 9C | 4C,9H | 3C,7G | 1H |
| Cheatgrass | 9C | 3C,9G | 5G | 4G |
| Cocklebur | 10C | 10C | 3C,9G | 2C,5G |
| Corn | 10C | 4U,9G | 4U,9G | 9G |
| Cotton | 5C,9G | 3C,9H | 3C,9H | 3C,6H |
| Crabgrass | 9C | 2C,8G | 4C,9G | 6G |
| Giant foxtail | 9C | 4C,9G | 4C,9G | 2C,7G |
| Morningglory | 10C | 3C,8H | 4C,9G | 4C,8H |
| Nutsedge | - | 4C,9G | 3G | 0 |
| Rice | 5C,9G | 5C,9G | 5C,9G | 5C,9G |
| Sorghum | 9C | 3U,9G | 2C,9G | 3C,7H |
| Soybean | 9C | 5C,9G | 4C,9G | 3C,7G |
| Sugar beet | 3C,7H | 0 | 0 | 0 |
| Velvetleaf | 4C,9H | 3C,7H | 2C,8H | 2H |
| Wheat | 9C | 2C,9G | 5C,9G | 9G |
| Wild oat | 2C,9G | 2C,7G | 0 | 0 |
| PREEMERGENCE | | | | |
| Barley | 3C,9G | 3C,7G | 3C,7G | 0 |
| Barnyardgrass | 9H | 0 | 0 | 0 |
| Cheatgrass | 3C,9G | 6G | 8G | 0 |
| Cocklebur | 9H | 1H | 2C | 5G |
| Corn | 3C,9H | 3C,3G | 3C,6G | 2G |
| Cotton | 2C,9G | 3G | 0 | 0 |
| Crabgrass | 5C,9H | 3C,9H | 7G | 2G |
| Giant foxtail | 9H | 6G | 6G | 2G |
| Morningglory | 9G | 8H | 8H | 4G |
| Nutsedge | 8G | 0 | 0 | 0 |
| Rice | 9H | 3C,8G | 9H | 8H |
| Sorghum | 3C,9G | 3C,7G | 3C,8H | 2C |
| Soybean | 9H | 3C,8H | 3C,6G | 2G |
| Sugar beet | 5G | 0 | 0 | 0 |
| Velvetleaf | 4C,8H | 0 | 0 | 0 |
| Wheat | 3C,9G | 8G | 8G | 5G |
| Wild oat | 2C,8G | 2C,5G | 0 | 0 |

Table A

| | Cmpd 13 | | Cmpd 14 | |
|---|---|---|---|---|
| RATE (g/ha) | 50 | 10 | 50 | 10 |
| POSTEMERGENCE | | | | |
| Barley | 8G | 6G | 5G | 2G |
| Barnyardgrass | 9C | 4C,9H | 3C,6H | 3C,8H |
| Cheatgrass | 9C | 7G | 7G | 5G |
| Cocklebur | 10C | 10C | 3C,7G | 2C,3H |
| Corn | 10C | 10C | 9C | 2C,8G |
| Cotton | 4C,9G | 4C,9G | 3C,3H | 3G |
| Crabgrass | 10C | 9C | 2C,5G | - |
| Giant foxtail | 10C | 4C,9G | 4C,9G | 3C,9G |
| Morningglory | 9C | 4C,8G | 3C,8H | 4C,8H |
| Nutsedge | 10C | 3C,7G | - | 0 |
| Rice | 9C | 9C | 4C,9G | 3C,8G |
| Sorghum | 9C | 9C | 3C,8G | 3C,7G |
| Soybean | 5C,9G | 4C,9G | 3C,9G | 2C,8G |
| Sugar beet | 7G | 5G | 3C,3H | - |
| Velvetleaf | 4C,9G | 4C,8H | 3C,7G | 7G |
| Wheat | 4C,9G | 8G | 8G | 2C,8G |
| Wild oat | 4G | 0 | 2G | 0 |
| PREEMERGENCE | | | | |
| Barley | 3C,7G | 3C,3G | 1C | 0 |
| Barnyardgrass | 7H | 4G | 1C | 0 |
| Cheatgrass | 8G | 0 | 2G | 0 |
| Cocklebur | 8H | 2C | 0 | 0 |
| Corn | 3C,9G | 3C,7H | 2C,4G | 0 |
| Cotton | 8G | 2C,2H | 0 | 0 |
| Crabgrass | 6G | 0 | 4G | 0 |
| Giant foxtail | 9H | 7H | 3C,7G | 0 |
| Morningglory | 8H | 8H | 0 | 0 |
| Nutsedge | 0 | 0 | 0 | 0 |
| Rice | 10E | 3C,8G | 2C,3G | 0 |
| Sorghum | 3C,9H | 3C,7G | 3C,6G | 0 |
| Soybean | 3C,8G | 3C,6H | 2C,4G | 0 |
| Sugar beet | 0 | 2H | 2H | 0 |
| Velvetleaf | 3C,5H | 2C,3H | 1H | 0 |
| Wheat | 8G | 3C,7G | 5G | 0 |
| Wild oat | 2C,4G | 0 | 0 | 0 |

Table A

| | Cmpd 15 | |
|---|---|---|
| RATE (g/ha) | 50 | 10 |
| POSTEMERGENCE | | |
| Barley | 9G | 2C,9G |
| Barnyardgrass | 4C,9H | 3C,7H |
| Cheatgrass | 8G | 8G |
| Cocklebur | 5C,9H | 3C,8H |
| Corn | 10C | 4C,9G |
| Cotton | 3C,5G | 3G |
| Crabgrass | 5C,9G | 7G |
| Giant foxtail | 5C,9H | 2C,8G |
| Morningglory | 4C,8H | 3C,8G |
| Nutsedge | 2C,7G | 3G |
| Rice | 5C,9G | 4C,9G |
| Sorghum | 5C,9G | 4C,9G |
| Soybean | 4C,9G | 3C,6H |
| Sugar beet | 7G | 3H |
| Velvetleaf | 7G | 3G |
| Wheat | 9G | 4C,9G |
| Wild oat | 3C,4G | 3G |
| PREEMERGENCE | | |
| Barley | 0 | 0 |
| Barnyardgrass | 2G | 0 |
| Cheatgrass | 4G | 0 |
| Cocklebur | 0 | 0 |
| Corn | 3C,7G | 0 |
| Cotton | 0 | 0 |
| Crabgrass | 2G | 0 |
| Giant foxtail | 3G | 0 |
| Morningglory | 0 | 0 |
| Nutsedge | 0 | 0 |
| Rice | 0 | 0 |
| Sorghum | 2C,3G | 0 |
| Soybean | 0 | 0 |
| Sugar beet | 0 | 0 |
| Velvetleaf | 0 | 0 |
| Wheat | 3G | 0 |
| Wild oat | 1C | 0 |

TEST B

Seeds of barley (Hordeum vulgare), barnyardgrass (Echinochloa crus-galli), blackgrass (Alopecurus myosuroides), cheatgrass (Bromus secalinus) or downy brome (Bromus tectorum), chickweed (Stellaria media), cocklebur (Xanthium pensylvanicum), corn (Zea mays), cotton (Gossypium hirsutum), crabgrass (Digitaria spp.), giant foxtail (Setaria faberi), green foxtail (Setaria viridis), jimsonweed (Datura stramonium), johnsongrass (Sorghum halepense), lambsquarters (Chenopodium album), morningglory (Ipomoea spp.), rape (Brassica napus), rice (Oryza sativa), sicklepod (Cassia obtusifolia), soybean (Glycine max), sugar beet (Beta vulgaris), teaweed (Sida spinosa), velvetleaf (Abutilon theophrasti), wheat (Triticum aestivum), wild buckwheat (Polygonum convolvulus), and wild oat (Avena fatua) and purple nutsedge (Cyperus rotundus) tubers were planted and treated preemergence with test chemicals dissolved in a non-phytotoxic solvent. At the same time, these crop and weed species were also treated with postemergence applications of test chemicals. Plants ranged in height from two to eighteen cm (two to three leaf stage) for postemergence treatments. Treated plants and controls were maintained in a greenhouse for approximately 24 days, after which all species were compared to controls and visually evaluated. The ratings, summarized in Table B, are

based on a scale of 0 to 100 where 0 is no effect and 100 is complete control. A dash (-) response means no test result.

Table B

| | Cmpd 1 | | | |
|---|---|---|---|---|
| RATE (g/ha) | 62 | 16 | 4 | 1 |
| POSTEMERGENCE | | | | |
| Barley | 90 | 90 | 60 | 30 |
| Barnyardgrass | 70 | 40 | 20 | 0 |
| Blackgrass | 90 | 80 | 50 | 30 |
| Cheatgrass | 80 | 70 | 50 | 30 |
| Chickweed | 100 | 80 | 50 | 30 |
| Cocklebur | 100 | 40 | 30 | 0 |
| Corn | 100 | 90 | 60 | 30 |
| Cotton | 70 | 30 | 0 | 0 |
| Crabgrass | 80 | 50 | 30 | 0 |
| Downy Brome | - | - | - | - |
| Giant foxtail | 100 | 80 | 50 | 30 |
| Green foxtail | 80 | 80 | 50 | 30 |
| Jimsonweed | 80 | 70 | 40 | 20 |
| Johnsongrass | 90 | 100 | 60 | 30 |
| Lambsquarters | 80 | 30 | 0 | 0 |
| Morningglory | 90 | 60 | 30 | 0 |
| Nutsedge | 40 | 0 | 0 | 0 |
| Rape | 0 | 0 | 0 | 0 |
| Rice | 70 | 70 | 50 | 30 |
| Sicklepod | 100 | 80 | 50 | 30 |
| Soybean | 100 | 60 | 40 | 20 |
| Sugar beet | 0 | 0 | 0 | 0 |
| Teaweed | 90 | 30 | 20 | 0 |
| Velvetleaf | 90 | 70 | 50 | 30 |
| Wheat | 100 | 60 | 30 | 0 |
| Wild buckwheat | 80 | 50 | 30 | 0 |
| Wild oat | 100 | 70 | 50 | 30 |

Table B

| Cmpd 1 | | | | |
|---|---|---|---|---|
| RATE (g/ha) | 250 | 62 | 16 | 4 |
| PREEMERGENCE | | | | |
| Barley | 50 | 50 | 20 | 0 |
| Barnyardgrass | 80 | 50 | 30 | 20 |
| Blackgrass | 90 | 40 | 40 | 20 |
| Cheatgrass | 80 | 70 | 50 | 40 |
| Chickweed | 100 | 70 | 40 | 30 |
| Cocklebur | 70 | 30 | 60 | 50 |
| Corn | 90 | 90 | 30 | 0 |
| Cotton | 30 | 10 | 30 | - |
| Crabgrass | 90 | 90 | 50 | 40 |
| Downy Brome | - | - | - | - |
| Giant foxtail | 100 | 80 | 30 | 0 |
| Green foxtail | 100 | 90 | 40 | 0 |
| Jimsonweed | 80 | 90 | 30 | 0 |
| Johnsongrass | 90 | 80 | 0 | 0 |
| Lambsquarters | 90 | 90 | 0 | 0 |
| Morningglory | 70 | 30 | 60 | 40 |
| Nutsedge | 60 | 30 | - | 20 |
| Rape | 20 | 10 | 0 | 0 |
| Rice | 100 | 80 | 30 | 20 |
| Sicklepod | - | 80 | - | 0 |
| Soybean | 40 | 10 | 20 | 10 |
| Sugar beet | 80 | 20 | 70 | 50 |
| Teaweed | 80 | 80 | 30 | - |
| Velvetleaf | 90 | 100 | 30 | - |
| Wheat | 70 | 60 | 0 | 0 |
| Wild buckwheat | 90 | 50 | 30 | - |
| Wild oat | 70 | 50 | 40 | 30 |

Table B

| | Cmpd 2 | | | |
|---|---|---|---|---|
| RATE (g/ha) | 500 | 250 | 62 | 16 |
| POSTEMERGENCE | | | | |
| Barley | 70 | 60 | 50 | 30 |
| Barnyardgrass | 60 | 50 | 0 | 0 |
| Blackgrass | 100 | 80 | 70 | 60 |
| Cheatgrass | 90 | 70 | 50 | 30 |
| Chickweed | 70 | 50 | 30 | 0 |
| Cocklebur | 100 | 70 | 50 | 30 |
| Corn | 100 | 100 | 100 | 100 |
| Cotton | 60 | 40 | 20 | 0 |
| Crabgrass | 100 | 90 | 70 | 50 |
| Downy Brome | - | - | - | - |
| Giant foxtail | 100 | 100 | 80 | 70 |
| Green foxtail | 100 | 100 | 70 | 50 |
| Jimsonweed | 80 | 70 | 50 | 30 |
| Johnsongrass | 100 | 80 | 70 | 50 |
| Lambsquarters | 70 | 50 | 30 | 0 |
| Morningglory | 50 | 30 | 20 | 0 |
| Nutsedge | 50 | 30 | 0 | 0 |
| Rape | 0 | 0 | 0 | 0 |
| Rice | 100 | 90 | 70 | 50 |
| Sicklepod | 90 | 70 | 50 | 30 |
| Soybean | 100 | 90 | 70 | 60 |
| Sugar beet | 0 | 0 | 0 | 0 |
| Teaweed | 90 | 80 | 70 | 50 |
| Velvetleaf | 100 | 90 | 70 | 50 |
| Wheat | 90 | 70 | 50 | 30 |
| Wild buckwheat | 60 | 30 | 0 | 0 |
| Wild oat | 100 | 90 | 70 | 60 |

Table B

| | Cmpd 2 | |
|---|---|---|
| RATE (g/ha) | 500 | 250 |
| PREEMERGENCE | | |
| Barley | 60 | 40 |
| Barnyardgrass | 100 | 100 |
| Blackgrass | 90 | 80 |
| Cheatgrass | 90 | 70 |
| Chickweed | 70 | 50 |
| Cocklebur | 50 | 30 |
| Corn | 100 | 90 |
| Cotton | 30 | 0 |
| Crabgrass | 95 | 90 |
| Downy Brome | - | - |
| Giant foxtail | - | - |
| Green foxtail | 95 | 90 |
| Jimsonweed | 90 | 80 |
| Johnsongrass | 90 | 80 |
| Lambsquarters | 100 | 100 |
| Morningglory | 90 | 70 |
| Nutsedge | 90 | 60 |
| Rape | 0 | 0 |
| Rice | 100 | 100 |
| Sicklepod | 100 | 100 |
| Soybean | 60 | 40 |
| Sugar beet | 0 | 0 |
| Teaweed | 90 | 60 |
| Velvetleaf | 100 | 100 |
| Wheat | 90 | 60 |
| Wild buckwheat | 80 | 50 |
| Wild oat | 90 | 70 |

Table B

| | Cmpd 3 | | | |
|---|---|---|---|---|
| RATE (g/ha) | 62 | 16 | 4 | 1 |
| POSTEMERGENCE | | | | |
| Barley | 80 | 50 | 20 | 0 |
| Barnyardgrass | 90 | 60 | 40 | 0 |
| Blackgrass | 80 | 70 | 40 | 30 |
| Cheatgrass | 70 | 40 | 30 | 0 |
| Chickweed | 100 | 90 | 50 | 30 |
| Cocklebur | 100 | 80 | 30 | 0 |
| Corn | 100 | 90 | 70 | 20 |
| Cotton | 90 | 20 | 0 | 0 |
| Crabgrass | 60 | 30 | 20 | 0 |
| Downy Brome | - | - | - | - |
| Giant foxtail | 80 | 40 | 30 | 0 |
| Green foxtail | 80 | 60 | 30 | 0 |
| Jimsonweed | 90 | 70 | 50 | 40 |
| Johnsongrass | 95 | 70 | 40 | 0 |
| Lambsquarters | 100 | 80 | 60 | 30 |
| Morningglory | 100 | 20 | 0 | 0 |
| Nutsedge | 60 | 40 | 30 | 20 |
| Rape | 10 | 0 | 0 | 0 |
| Rice | 100 | 90 | 80 | 40 |
| Sicklepod | 100 | 50 | 20 | 0 |
| Soybean | 90 | 70 | 30 | 0 |
| Sugar beet | 90 | 20 | 0 | 0 |
| Teaweed | 50 | 20 | 0 | 0 |
| Velvetleaf | 90 | 60 | 30 | 0 |
| Wheat | 70 | 50 | 30 | 0 |
| Wild buckwheat | 60 | 30 | 0 | 0 |
| Wild oat | 90 | 70 | 30 | 0 |

Table B

| | Cmpd 3 | | |
|---|---|---|---|
| RATE (g/ha) | 250 | 62 | 16 |
| PREEMERGENCE | | | |
| Barley | 30 | 0 | 0 |
| Barnyardgrass | 60 | 40 | 0 |
| Blackgrass | 90 | 80 | 70 |
| Cheatgrass | 90 | 60 | 30 |
| Chickweed | 70 | 50 | 30 |
| Cocklebur | 50 | 30 | 0 |
| Corn | 50 | 0 | 0 |
| Cotton | 30 | 0 | 0 |
| Crabgrass | 90 | 60 | 30 |
| Downy Brome | - | - | - |
| Giant foxtail | 90 | 60 | 30 |
| Green foxtail | 100 | 60 | 30 |
| Jimsonweed | 80 | 50 | 30 |
| Johnsongrass | 90 | 80 | 60 |
| Lambsquarters | 100 | 95 | 90 |
| Morningglory | 90 | 70 | 60 |
| Nutsedge | 30 | 0 | 0 |
| Rape | 0 | 0 | 0 |
| Rice | 100 | 60 | 40 |
| Sicklepod | 50 | 30 | 0 |
| Soybean | 0 | 0 | 0 |
| Sugar beet | 100 | 60 | 30 |
| Teaweed | 50 | 0 | 0 |
| Velvetleaf | 60 | 30 | 0 |
| Wheat | 60 | 30 | 0 |
| Wild buckwheat | 50 | 30 | 0 |
| Wild oat | 60 | 30 | 0 |

Table B

| | Cmpd 6 | | | |
|---|---|---|---|---|
| RATE (g/ha) | 250 | 62 | 16 | 4 |
| POSTEMERGENCE | | | | |
| Barley | 90 | 70 | 30 | 30 |
| Barnyardgrass | 80 | 70 | 40 | 0 |
| Blackgrass | 90 | 70 | 50 | 30 |
| Cheatgrass | - | - | - | - |
| Chickweed | 100 | 90 | 80 | 50 |
| Cocklebur | 100 | 70 | 0 | 0 |
| Corn | 80 | 70 | 30 | 30 |
| Cotton | 90 | 70 | 30 | 0 |
| Crabgrass | 100 | 80 | 70 | 30 |
| Downy Brome | 100 | 70 | 50 | 30 |
| Giant foxtail | 90 | 60 | 30 | 0 |
| Green foxtail | 80 | 70 | 30 | 0 |
| Jimsonweed | 60 | 50 | 40 | - |
| Johnsongrass | 100 | 90 | 40 | 0 |
| Lambsquarters | 100 | 90 | 70 | 50 |
| Morningglory | 100 | 100 | 50 | 40 |
| Nutsedge | 50 | 0 | 0 | 0 |
| Rape | 70 | 50 | 30 | 0 |
| Rice | 100 | 80 | 60 | 50 |
| Sicklepod | 100 | 90 | 0 | 0 |
| Soybean | 100 | 100 | 60 | 20 |
| Sugar beet | 90 | 70 | 50 | 30 |
| Teaweed | 80 | 70 | 30 | - |
| Velvetleaf | 95 | 90 | 80 | 60 |
| Wheat | 70 | 60 | 60 | 30 |
| Wild buckwheat | 90 | 70 | 30 | 0 |
| Wild oat | 70 | 50 | 30 | 0 |

Table B

| | Cmpd 6 | | |
|---|---|---|---|
| RATE (g/ha) | 250 | 62 | 16 |
| PREEMERGENCE | | | |
| Barley | 70 | 30 | 0 |
| Barnyardgrass | 80 | 40 | 30 |
| Blackgrass | 90 | 80 | 60 |
| Cheatgrass | - | - | - |
| Chickweed | 95 | 90 | 70 |
| Cocklebur | 100 | 100 | 60 |
| Corn | 70 | 0 | 0 |
| Cotton | 60 | 40 | 0 |
| Crabgrass | 100 | 90 | 85 |
| Downy Brome | 90 | 50 | 30 |
| Giant foxtail | 100 | 80 | 30 |
| Green foxtail | 100 | 80 | 50 |
| Jimsonweed | 70 | 50 | 30 |
| Johnsongrass | 90 | 80 | 50 |
| Lambsquarters | 100 | 90 | 80 |
| Morningglory | 90 | 40 | 30 |
| Nutsedge | 0 | 0 | 0 |
| Rape | 0 | 0 | 0 |
| Rice | 90 | 80 | 70 |
| Sicklepod | 80 | 60 | 0 |
| Soybean | 40 | 20 | 0 |
| Sugar beet | 90 | 80 | 60 |
| Teaweed | 70 | 60 | 30 |
| Velvetleaf | 100 | 60 | 30 |
| Wheat | 70 | 60 | 30 |
| Wild buckwheat | 60 | 30 | 0 |
| Wild oat | 70 | 50 | 30 |

Table B

Cmpd 10

| RATE (g/ha) | 62 | 16 | 4 | 1 |
|---|---|---|---|---|
| POSTEMERGENCE | | | | |
| Barley | 90 | 90 | 80 | 30 |
| Barnyardgrass | 90 | 90 | 70 | 30 |
| Blackgrass | 90 | 80 | 50 | 50 |
| Cheatgrass | - | - | - | - |
| Chickweed | 90 | 90 | 70 | 50 |
| Cocklebur | 60 | 20 | 0 | 0 |
| Corn | 100 | 90 | 70 | 0 |
| Cotton | 80 | 60 | 40 | 30 |
| Crabgrass | 90 | 80 | 40 | 30 |
| Downy Brome | 90 | 80 | 70 | 20 |
| Giant foxtail | 90 | 90 | 60 | 20 |
| Green foxtail | 90 | 90 | 40 | 0 |
| Jimsonweed | 90 | 90 | 50 | 50 |
| Johnsongrass | 100 | 90 | 80 | 50 |
| Lambsquarters | 90 | 90 | 40 | 0 |
| Morningglory | 90 | 70 | 70 | 50 |
| Nutsedge | 0 | 0 | 0 | 0 |
| Rape | 0 | 0 | 0 | 0 |
| Rice | 100 | 100 | 100 | 80 |
| Sicklepod | 90 | 80 | 30 | 0 |
| Soybean | 90 | 90 | 80 | 20 |
| Sugar beet | 100 | 100 | 60 | 0 |
| Teaweed | 50 | 30 | 0 | 0 |
| Velvetleaf | 100 | 100 | 90 | 70 |
| Wheat | 90 | 90 | 80 | 30 |
| Wild buckwheat | 90 | 50 | 50 | 0 |
| Wild oat | 90 | 40 | 30 | 0 |

Table B

Cmpd 10

| RATE (g/ha) | 62 | 16 | 4 | 1 |
|---|---|---|---|---|
| PREEMERGENCE | | | | |
| Barley | 90 | 60 | 20 | 0 |
| Barnyardgrass | 90 | 90 | 40 | 30 |
| Blackgrass | 90 | 70 | 50 | 30 |
| Cheatgrass | - | - | - | - |
| Chickweed | 90 | 80 | 70 | 50 |
| Cocklebur | 30 | 0 | 0 | 0 |
| Corn | 90 | 50 | 30 | 20 |
| Cotton | 90 | 80 | 20 | 0 |
| Crabgrass | 100 | 100 | 90 | 50 |
| Downy Brome | 80 | 80 | 50 | 30 |
| Giant foxtail | 90 | 90 | 80 | 50 |
| Green foxtail | 100 | 90 | 70 | 50 |
| Jimsonweed | 100 | 100 | 50 | 0 |
| Johnsongrass | 100 | 90 | 60 | 50 |
| Lambsquarters | 90 | 80 | 30 | 20 |
| Morningglory | 100 | 90 | 40 | 30 |
| Nutsedge | 50 | 0 | 0 | 0 |
| Rape | 0 | 0 | 0 | 0 |
| Rice | 90 | 90 | 70 | 40 |
| Sicklepod | 90 | 80 | 30 | 0 |
| Soybean | 70 | 30 | 0 | 0 |
| Sugar beet | 90 | 80 | 30 | 0 |
| Teaweed | 90 | 50 | 0 | 0 |
| Velvetleaf | 100 | 100 | 30 | 0 |
| Wheat | 90 | 40 | 30 | 0 |
| Wild buckwheat | 90 | 90 | 0 | 0 |
| Wild oat | 50 | 40 | 30 | 0 |

Table B

Cmpd 11

| RATE (g/ha) | 62 | 16 | 4 | 1 |
|---|---|---|---|---|
| POSTEMERGENCE | | | | |
| Barley | 70 | 50 | 30 | 0 |
| Barnyardgrass | 80 | 70 | 30 | 0 |
| Blackgrass | 100 | 100 | 100 | 60 |
| Cheatgrass | - | - | - | - |
| Chickweed | 90 | 80 | 70 | 60 |
| Cocklebur | 90 | 70 | 60 | 30 |
| Corn | 90 | 80 | 70 | 0 |
| Cotton | 70 | 60 | 20 | 0 |
| Crabgrass | 100 | 100 | 70 | 30 |
| Downy Brome | 90 | 80 | 50 | 30 |
| Giant foxtail | 100 | 90 | 70 | 30 |
| Green foxtail | 100 | 80 | 50 | 30 |
| Jimsonweed | 100 | 80 | 70 | 40 |
| Johnsongrass | 90 | 80 | 30 | 0 |
| Lambsquarters | 90 | 60 | 50 | 30 |
| Morningglory | 90 | 70 | 50 | 0 |
| Nutsedge | 90 | 60 | 0 | 0 |
| Rape | 100 | 100 | 90 | 70 |
| Rice | 90 | 80 | 70 | 60 |
| Sicklepod | 80 | 60 | 30 | 0 |
| Soybean | 100 | 90 | 70 | 30 |
| Sugar beet | 30 | 0 | 0 | 0 |
| Teaweed | 90 | 50 | 30 | 0 |
| Velvetleaf | 90 | 80 | 70 | 30 |
| Wheat | 90 | 80 | 70 | 50 |
| Wild buckwheat | 90 | 70 | 60 | 40 |
| Wild oat | 60 | 50 | 30 | 0 |

Table B

Cmpd 11

| RATE (g/ha) | 62 | 16 | 4 | 1 |
|---|---|---|---|---|
| PREEMERGENCE | | | | |
| Barley | 50 | 0 | 0 | 0 |
| Barnyardgrass | 100 | 60 | 30 | 0 |
| Blackgrass | 70 | 50 | 30 | 0 |
| Cheatgrass | - | - | - | - |
| Chickweed | 90 | 70 | 50 | 30 |
| Cocklebur | 100 | 80 | 50 | 30 |
| Corn | 50 | 20 | 0 | 0 |
| Cotton | 50 | 30 | 0 | 0 |
| Crabgrass | 90 | 60 | 30 | 0 |
| Downy Brome | 80 | 50 | 30 | 0 |
| Giant foxtail | 90 | 80 | 70 | 30 |
| Green foxtail | 100 | 100 | 90 | 30 |
| Jimsonweed | 90 | 80 | 70 | 60 |
| Johnsongrass | 90 | 80 | 60 | 30 |
| Lambsquarters | 100 | 90 | 50 | 0 |
| Morningglory | 95 | 90 | 80 | 50 |
| Nutsedge | 90 | 50 | 30 | 0 |
| Rape | 90 | 40 | 0 | 0 |
| Rice | 90 | 80 | 60 | 50 |
| Sicklepod | 90 | 70 | 60 | 50 |
| Soybean | 70 | 30 | 0 | 0 |
| Sugar beet | 70 | 50 | 30 | 20 |
| Teaweed | 70 | 50 | 40 | 30 |
| Velvetleaf | 100 | 70 | 50 | 30 |
| Wheat | 70 | 50 | 30 | 0 |
| Wild buckwheat | 90 | 80 | 70 | 50 |
| Wild oat | 50 | 0 | 0 | 0 |

Table B

| | Cmpd 12 | | | |
|---|---|---|---|---|
| RATE (g/ha) | 250 | 62 | 16 | 4 |
| POSTEMERGENCE | | | | |
| Barley | 80 | 40 | 20 | 0 |
| Barnyardgrass | 70 | 40 | 20 | 0 |
| Blackgrass | 100 | 80 | 70 | 50 |
| Cheatgrass | - | - | - | - |
| Chickweed | 80 | 50 | 30 | 0 |
| Cocklebur | 80 | 70 | 30 | 0 |
| Corn | 100 | 70 | 70 | 20 |
| Cotton | 70 | 40 | 30 | 0 |
| Crabgrass | 70 | 20 | 0 | 0 |
| Downy Brome | 100 | 100 | 80 | 70 |
| Giant foxtail | 80 | 80 | 40 | 0 |
| Green foxtail | 100 | 70 | 40 | 0 |
| Jimsonweed | 80 | 40 | 30 | 0 |
| Johnsongrass | 80 | 20 | 0 | 0 |
| Lambsquarters | 70 | 50 | 0 | 0 |
| Morningglory | 80 | 80 | 80 | 0 |
| Nutsedge | 80 | 0 | 0 | 0 |
| Rape | 100 | 80 | 80 | 50 |
| Rice | 100 | 100 | 90 | 80 |
| Sicklepod | 30 | 0 | 0 | 0 |
| Soybean | 90 | 80 | 70 | 40 |
| Sugar beet | 80 | 60 | 40 | 0 |
| Teaweed | 80 | 30 | 0 | 0 |
| Velvetleaf | 70 | 50 | 0 | 0 |
| Wheat | 90 | 90 | 80 | 30 |
| Wild buckwheat | 90 | 80 | 30 | 0 |
| Wild oat | 50 | 30 | 20 | 0 |

Table B

Cmpd 12

| RATE (g/ha) | 250 | 62 | 16 | 4 |
|---|---|---|---|---|
| PREEMERGENCE | | | | |
| Barley | 90 | 50 | 30 | 0 |
| Barnyardgrass | 70 | 30 | 0 | 0 |
| Blackgrass | 80 | 80 | 50 | 30 |
| Cheatgrass | - | - | - | - |
| Chickweed | 40 | 20 | 0 | 0 |
| Cocklebur | 80 | 70 | 50 | 30 |
| Corn | 100 | 40 | 20 | 0 |
| Cotton | 80 | 50 | 20 | 0 |
| Crabgrass | 100 | 80 | 30 | 0 |
| Downy Brome | 100 | 100 | 70 | 30 |
| Giant foxtail | 90 | 80 | 40 | 30 |
| Green foxtail | 100 | 90 | 70 | 30 |
| Jimsonweed | 40 | 20 | 0 | 0 |
| Johnsongrass | 100 | 90 | 30 | 0 |
| Lambsquarters | 100 | 100 | 50 | 20 |
| Morningglory | 90 | 90 | 30 | 0 |
| Nutsedge | 100 | 100 | 20 | 0 |
| Rape | 90 | 50 | 30 | 0 |
| Rice | 100 | 100 | 90 | 80 |
| Sicklepod | 80 | 50 | 20 | 0 |
| Soybean | 90 | 60 | 20 | 0 |
| Sugar beet | 50 | 20 | 0 | 0 |
| Teaweed | 80 | 30 | 0 | 0 |
| Velvetleaf | 50 | 20 | 0 | 0 |
| Wheat | 90 | 90 | 50 | 40 |
| Wild buckwheat | 90 | 30 | 20 | 0 |
| Wild oat | 60 | 40 | 30 | 0 |

Table B

Cmpd 13

| RATE (g/ha) | 62 | 16 | 4 | 1 |
|---|---|---|---|---|
| POSTEMERGENCE | | | | |
| Barley | 60 | 30 | 30 | 0 |
| Barnyardgrass | 100 | 80 | 50 | 30 |
| Blackgrass | 100 | 90 | 80 | 50 |
| Cheatgrass | - | - | - | - |
| Chickweed | 90 | 30 | 0 | 0 |
| Cocklebur | 100 | 100 | 90 | 70 |
| Corn | 100 | 100 | 90 | 85 |
| Cotton | 90 | 90 | 60 | 40 |
| Crabgrass | 90 | 60 | 50 | 40 |
| Downy Brome | 100 | 100 | 90 | 80 |
| Giant foxtail | 100 | 90 | 80 | 50 |
| Green foxtail | 100 | 100 | 70 | 60 |
| Jimsonweed | 80 | 80 | 60 | 50 |
| Johnsongrass | 100 | 90 | 80 | 80 |
| Lambsquarters | 60 | 30 | 0 | - |
| Morningglory | 100 | 100 | 80 | 60 |
| Nutsedge | 100 | 40 | 30 | 0 |
| Rape | 100 | 100 | 100 | 90 |
| Rice | 100 | 100 | 95 | 90 |
| Sicklepod | 100 | 40 | 40 | 10 |
| Soybean | 100 | 100 | 80 | 50 |
| Sugar beet | 80 | 0 | 0 | 0 |
| Teaweed | 80 | 60 | 50 | 40 |
| Velvetleaf | 80 | 80 | 70 | 50 |
| Wheat | 100 | 90 | 80 | 50 |
| Wild buckwheat | 90 | 60 | 60 | 0 |
| Wild oat | 60 | 30 | 30 | 0 |

Table B

Cmpd 13

| RATE (g/ha) | 250 | 62 | 16 | 4 |
|---|---|---|---|---|
| PREEMERGENCE | | | | |
| Barley | 80 | 70 | 60 | 0 |
| Barnyardgrass | 90 | 70 | 30 | 0 |
| Blackgrass | 100 | 90 | 70 | 30 |
| Cheatgrass | - | - | - | - |
| Chickweed | 90 | 70 | 50 | 0 |
| Cocklebur | 90 | 80 | - | 60 |
| Corn | 100 | 60 | 20 | 0 |
| Cotton | 60 | 50 | 20 | 0 |
| Crabgrass | 100 | 90 | 70 | 50 |
| Downy Brome | 90 | 80 | 50 | 0 |
| Giant foxtail | 90 | 80 | 60 | 30 |
| Green foxtail | 100 | 100 | 100 | 90 |
| Jimsonweed | 90 | 60 | 30 | 30 |
| Johnsongrass | 90 | 80 | 70 | 60 |
| Lambsquarters | 95 | 90 | 80 | 70 |
| Morningglory | 90 | 80 | 60 | 50 |
| Nutsedge | 80 | 40 | 20 | 0 |
| Rape | 90 | 80 | 70 | 40 |
| Rice | 100 | 90 | 80 | 70 |
| Sicklepod | 90 | 70 | 60 | 50 |
| Soybean | 70 | 60 | 20 | 0 |
| Sugar beet | 70 | 60 | 50 | 40 |
| Teaweed | 90 | 60 | 50 | 30 |
| Velvetleaf | 100 | 80 | 70 | 40 |
| Wheat | 90 | 70 | 50 | 30 |
| Wild buckwheat | 90 | 80 | 70 | 60 |
| Wild oat | 50 | 40 | 30 | 0 |

TEST C

Seeds selected from crop and weed species consisting of barley (Hordeum vulgare), black nightshade (Solanum nigrum), blackgrass (Alopecurus myosuroides), bluegrass (Poa annua), catchweed bedstraw (Galium aparine), chickweed (Stellaria media), green foxtail (Setaria viridis), Italian ryegrass (Lolium multiflorum), knotweed (Polygonum aviculare), kochia (Kochia scoparia), lambsquarters (Chenopodium album), mustard (Brassica spp.), Persian speedwell (Veronica persica), pigweed (Amaranthus retroflexus), rape (Brassica napus), scentless chamomille (Matricaria inodora), smartweed (Polygonum persicaria), sugar beet (Beta vulgaris), viola (Viola arvensis), wheat (Triticum aestivum), wild buckwheat (Polygonum convolvulus), wild oat (Avena fatua) and wild radish (Raphanus raphanistrum) were planted and treated preemergence with test chemicals dissolved in a non-phytotoxic solvent. Selected species from this list of crops and weeds were also treated with postemergence applications of test chemicals. Plants ranged in height from two to twenty cm (two to three leaf stage) for postemergence treatments. Treated plants and controls were maintained in a greenhouse for approximately 24 days, after which all species were compared to controls and visually evaluated. Response ratings, summarized in Table C, are based on a scale of 0 to 100 where 0 is no effect and 100 is complete control. A dash (-) response means no test result.

Table C

| | Cmpd 1 | | | | |
|---|---|---|---|---|---|
| RATE (g/ha) | 250 | 125 | 64 | 32 | 16 |
| POSTEMERGENCE | | | | | |
| Barley | 90 | 90 | 100 | 80 | 80 |
| Black nightshade | 100 | 100 | 100 | 100 | 100 |
| Blackgrass | 100 | 100 | 100 | 100 | 100 |
| Bluegrass | 100 | 100 | 90 | 80 | 80 |
| Catchweed bedstraw | 100 | 100 | 90 | 90 | 100 |
| Chickweed | 100 | 90 | 80 | 80 | 50 |
| Green foxtail | 100 | 100 | 100 | 80 | 90 |
| Italian ryegrass | 100 | 100 | 100 | 90 | 100 |
| Kochia | 100 | 100 | 85 | 70 | 40 |
| Lambsquarters | 95 | 80 | 50 | 40 | 50 |
| Mustard | 100 | 100 | 100 | 100 | 100 |
| Persian speedwell | 100 | 85 | 85 | 75 | 75 |
| Pigweed | 100 | 100 | 100 | 100 | 100 |
| Scentless chamomille | 90 | 90 | 80 | 50 | 50 |
| Sugar beet | 50 | 30 | 20 | 10 | 10 |
| Wheat | 90 | 90 | 90 | 80 | 80 |
| Wild buckwheat | 100 | 100 | 85 | 80 | 70 |
| Wild oat | 90 | 90 | 100 | 90 | 80 |
| Wild radish | 100 | 60 | 50 | 50 | 50 |
| PREEMERGENCE | | | | | |
| Blackgrass | 80 | 70 | 70 | 30 | 30 |
| Catchweed bedstraw | 90 | 70 | 70 | 50 | 0 |
| Chickweed | 70 | 0 | 0 | 0 | 0 |
| Lambsquarters | 98 | - | 30 | 0 | 0 |
| Persian speedwell | 100 | 100 | 80 | 0 | 0 |
| Scentless chamomille | 80 | 80 | 80 | 80 | 70 |
| Sugar beet | 40 | 30 | 0 | 0 | 0 |
| Viola | 0 | 0 | 0 | 0 | 0 |
| Wheat | 60 | 50 | 50 | 50 | 30 |
| Wild buckwheat | 70 | 70 | 50 | 30 | 30 |
| Wild oat | 70 | 70 | 70 | 40 | 40 |

Table C

| | Cmpd 2 | |
|---|---|---|
| RATE (g/ha) | 125 | 64 |
| POSTEMERGENCE | | |
| Barley | 50 | 60 |
| Black nightshade | 40 | 40 |
| Blackgrass | 75 | 75 |
| Bluegrass | 80 | 80 |
| Catchweed bedstraw | 75 | 75 |
| Chickweed | 90 | 80 |
| Green foxtail | 100 | 80 |
| Italian ryegrass | 80 | 80 |
| Kochia | 75 | 30 |
| Lambsquarters | 60 | 20 |
| Mustard | 100 | 100 |
| Persian speedwell | 0 | 0 |
| Pigweed | 100 | 100 |
| Scentless chamomille | 50 | 50 |
| Smartweed | 40 | 0 |
| Sugar beet | 20 | 40 |
| Wheat | 70 | 50 |
| Wild buckwheat | 40 | 20 |
| Wild oat | 50 | 50 |
| Wild radish | 80 | 50 |
| PREEMERGENCE | | |
| Barley | 40 | 40 |
| Black nightshade | 0 | 0 |
| Blackgrass | 100 | 100 |
| Bluegrass | 80 | 90 |
| Catchweed bedstraw | 80 | 80 |
| Chickweed | 0 | 0 |
| Green foxtail | 100 | 75 |
| Italian ryegrass | 85 | 50 |
| Kochia | 40 | 20 |
| Lambsquarters | 0 | 0 |
| Mustard | 80 | 0 |
| Persian speedwell | 30 | 0 |
| Pigweed | 100 | 100 |
| Scentless chamomille | 100 | 100 |
| Sugar beet | 0 | 20 |
| Wheat | 40 | 20 |
| Wild buckwheat | 0 | 0 |
| Wild oat | 30 | 30 |
| Wild radish | 0 | 0 |

Table C

| Cmpd 3 | | | | |
|---|---|---|---|---|
| RATE (g/ha) | 64 | 32 | 16 | 8 |
| POSTEMERGENCE | | | | |
| Barley | 50 | 50 | 50 | 60 |
| Black nightshade | 40 | 40 | 20 | 0 |
| Blackgrass | 100 | 100 | 60 | 60 |
| Bluegrass | 50 | 50 | 20 | 0 |
| Catchweed bedstraw | 100 | 80 | - | - |
| Chickweed | 100 | 70 | 70 | 80 |
| Green foxtail | 20 | 0 | 0 | 0 |
| Italian ryegrass | 40 | 0 | 0 | 0 |
| Kochia | 100 | 0 | - | - |
| Lambsquarters | 20 | 0 | 0 | 0 |
| Mustard | 100 | 100 | 70 | 70 |
| Persian speedwell | 40 | 20 | 0 | 0 |
| Pigweed | 100 | 100 | 50 | 30 |
| Rape | 0 | 0 | 30 | 0 |
| Scentless chamomille | 60 | 0 | 0 | 0 |
| Smartweed | 100 | 80 | - | - |
| Sugar beet | 60 | 30 | 10 | 0 |
| Wheat | 60 | 60 | 80 | 40 |
| Wild buckwheat | 100 | 40 | 60 | 60 |
| Wild oat | 80 | 90 | 80 | 80 |
| Wild radish | 0 | 0 | 0 | 0 |
| PREEMERGENCE | | | | |
| Barley | 30 | 0 | | |
| Black nightshade | 20 | 0 | | |
| Blackgrass | 50 | 0 | | |
| Bluegrass | 20 | 0 | | |
| Catchweed bedstraw | 100 | 0 | | |
| Chickweed | 0 | 0 | | |
| Green foxtail | 70 | 0 | | |
| Italian ryegrass | 0 | 0 | | |
| Kochia | 0 | 0 | | |
| Lambsquarters | 0 | 0 | | |
| Mustard | 0 | 0 | | |
| Persian speedwell | 0 | 0 | | |
| Pigweed | 20 | 0 | | |
| Rape | 0 | 0 | | |
| Scentless chamomille | 0 | 0 | | |
| Smartweed | 100 | 100 | | |
| Sugar beet | 0 | 0 | | |
| Wheat | 20 | 0 | | |
| Wild buckwheat | 0 | 0 | | |
| Wild oat | 60 | 40 | | |
| Wild radish | 0 | 0 | | |

Table C

| | Cmpd 4 | | | |
|---|---|---|---|---|
| RATE (g/ha) | 250 | 125 | 64 | 32 |
| POSTEMERGENCE | | | | |
| Blackgrass | 90 | 50 | 50 | 20 |
| Catchweed bedstraw | 80 | 80 | 30 | 30 |
| Chickweed | 80 | 80 | 80 | 80 |
| Lambsquarters | 0 | 0 | 0 | 0 |
| Persian speedwell | 0 | 0 | 0 | 0 |
| Scentless chamomille | 90 | 90 | 90 | 50 |
| Sugar beet | 20 | 10 | 10 | 0 |
| Viola | 0 | 0 | 0 | 0 |
| Wheat | 50 | 50 | 30 | 20 |
| Wild buckwheat | 0 | 0 | 0 | 0 |
| Wild oat | 50 | 50 | 50 | 50 |
| PREEMERGENCE | | | | |
| Blackgrass | 100 | 90 | 90 | 90 |
| Catchweed bedstraw | 100 | 70 | 30 | 0 |
| Chickweed | 90 | 80 | 50 | 0 |
| Knotweed | 80 | 20 | 0 | 0 |
| Persian speedwell | 0 | 0 | 0 | 0 |
| Scentless chamomille | 100 | 90 | 90 | 90 |
| Sugar beet | 0 | 0 | 0 | 0 |
| Viola | 100 | 90 | 90 | 50 |
| Wheat | 90 | 70 | 40 | 0 |
| Wild buckwheat | 0 | 0 | 0 | 0 |
| Wild oat | 80 | 80 | 50 | 30 |

Table C

Cmpd 5

| RATE (g/ha) | 250 | 125 | 64 | 32 | 16 |
|---|---|---|---|---|---|
| POSTEMERGENCE | | | | | |
| Blackgrass | 100 | 90 | 90 | 50 | 50 |
| Catchweed bedstraw | 100 | 100 | 100 | 90 | 90 |
| Chickweed | 100 | 90 | 90 | 90 | 90 |
| Lambsquarters | 100 | 0 | 0 | 0 | 0 |
| Persian speedwell | 100 | 100 | 50 | 20 | 0 |
| Scentless chamomille | 100 | 100 | 100 | 100 | 100 |
| Sugar beet | 70 | 0 | 0 | 0 | 0 |
| Viola | 50 | 50 | 50 | 0 | 0 |
| Wheat | 70 | 50 | 50 | 30 | 30 |
| Wild buckwheat | 50 | - | 50 | 30 | 0 |
| Wild oat | 90 | 90 | 90 | 90 | 90 |
| PREEMERGENCE | | | | | |
| Blackgrass | 100 | 100 | 100 | 100 | |
| Catchweed bedstraw | 100 | 100 | 100 | 70 | |
| Chickweed | 100 | 90 | 90 | 30 | |
| Knotweed | 90 | 20 | 0 | 0 | |
| Persian speedwell | 100 | 100 | 50 | 50 | |
| Scentless chamomille | 100 | 100 | 100 | 90 | |
| Sugar beet | 0 | 0 | 0 | 0 | |
| Viola | 100 | 90 | 70 | 70 | |
| Wheat | 100 | 90 | 70 | 50 | |
| Wild buckwheat | 30 | 30 | 0 | 0 | |
| Wild oat | 90 | 90 | 70 | 30 | |

Table C

| | Cmpd 6 | | | | |
|---|---|---|---|---|---|
| RATE (g/ha) | 125 | 64 | 32 | 16 | 8 |
| POSTEMERGENCE | | | | | |
| Blackgrass | 100 | 90 | 70 | 0 | 0 |
| Catchweed bedstraw | 100 | 90 | - | - | 20 |
| Chickweed | 100 | 100 | 100 | 100 | 100 |
| Knotweed | 0 | 0 | 0 | 0 | 0 |
| Lambsquarters | 100 | 90 | 75 | 75 | 30 |
| Persian speedwell | 30 | 0 | 0 | 0 | 0 |
| Scentless chamomille | 100 | 70 | 70 | 20 | 0 |
| Sugar beet | 50 | 50 | 20 | 10 | 0 |
| Viola | 100 | 100 | 100 | 70 | 0 |
| Wheat | 50 | 50 | 50 | 30 | 30 |
| Wild buckwheat | 100 | 80 | 20 | 20 | 0 |
| Wild oat | 50 | 50 | 50 | 20 | 0 |
| PREEMERGENCE | | | | | |
| Blackgrass | 80 | 50 | 50 | 0 | |
| Catchweed bedstraw | 90 | 90 | 70 | 20 | |
| Chickweed | 50 | 30 | 0 | 0 | |
| Knotweed | 70 | 70 | 70 | 0 | |
| Lambsquarters | 100 | 70 | 30 | 30 | |
| Persian speedwell | 20 | 0 | 0 | 0 | |
| Scentless chamomille | 80 | 80 | 70 | 50 | |
| Sugar beet | 40 | 20 | 0 | 0 | |
| Viola | 50 | 50 | 30 | 0 | |
| Wheat | 70 | 30 | 0 | 0 | |
| Wild buckwheat | 0 | 0 | 0 | 0 | |
| Wild oat | 50 | 30 | 0 | 0 | |

Table C

| | Cmpd 7 | | | | |
|---|---|---|---|---|---|
| RATE (g/ha) | 125 | 64 | 32 | 16 | 8 |
| POSTEMERGENCE | | | | | |
| Blackgrass | 50 | 50 | 30 | 0 | 0 |
| Catchweed bedstraw | 70 | 70 | 50 | 0 | 0 |
| Chickweed | 70 | 70 | 70 | 70 | 30 |
| Lambsquarters | 60 | 60 | 40 | 20 | 0 |
| Persian speedwell | 80 | 70 | 60 | 60 | 20 |
| Sugar beet | 40 | 40 | 30 | 0 | 0 |
| Viola | 80 | 50 | 30 | 30 | 0 |
| Wheat | 50 | 30 | 30 | 20 | 0 |
| Wild buckwheat | 60 | 50 | 50 | 20 | 0 |
| Wild oat | 30 | 20 | 0 | 0 | 0 |
| PREEMERGENCE | | | | | |
| Blackgrass | 60 | 40 | 30 | 30 | |
| Catchweed bedstraw | 70 | 30 | 0 | 0 | |
| Chickweed | 80 | 70 | 50 | 0 | |
| Lambsquarters | 40 | 0 | 0 | 0 | |
| Persian speedwell | 50 | 30 | 0 | 0 | |
| Scentless chamomille | 80 | 70 | 30 | 30 | |
| Sugar beet | 80 | 40 | 0 | 0 | |
| Viola | 70 | 50 | 20 | 0 | |
| Wheat | 40 | 30 | 0 | 0 | |
| Wild buckwheat | 60 | 40 | 0 | 0 | |
| Wild oat | 40 | 20 | 20 | 0 | |

Table C

Cmpd 11

| RATE (g/ha) | 64 | 32 | 16 | 8 |
|---|---|---|---|---|
| POSTEMERGENCE | | | | |
| Blackgrass | 100 | 100 | 100 | 100 |
| Catchweed bedstraw | 100 | 100 | 100 | 50 |
| Chickweed | 100 | 100 | 80 | 80 |
| Lambsquarters | 100 | 100 | 100 | 100 |
| Persian speedwell | 0 | 0 | 0 | 0 |
| Scentless chamomille | 100 | 100 | 100 | 100 |
| Sugar beet | 100 | 30 | 10 | 0 |
| Viola | 100 | 100 | 100 | 70 |
| Wheat | 100 | 100 | 100 | 70 |
| Wild buckwheat | 90 | 90 | 70 | 70 |
| Wild oat | 100 | 100 | 70 | 70 |
| PREEMERGENCE | | | | |
| Blackgrass | 100 | 100 | | |
| Catchweed bedstraw | 100 | 100 | | |
| Chickweed | 100 | 100 | | |
| Knotweed | 0 | 0 | | |
| Persian speedwell | 0 | 0 | | |
| Scentless chamomille | 100 | 90 | | |
| Sugar beet | 90 | 80 | | |
| Viola | 100 | 100 | | |
| Wheat | 100 | 100 | | |
| Wild buckwheat | 50 | 20 | | |
| Wild oat | 80 | 30 | | |

Table C

| | Cmpd 12 | | | | |
|---|---|---|---|---|---|
| RATE (g/ha) | 64 | 32 | 16 | 8 | 4 |
| POSTEMERGENCE | | | | | |
| Blackgrass | 70 | 70 | 30 | 30 | 30 |
| Catchweed bedstraw | 50 | 30 | 0 | 0 | 0 |
| Chickweed | 30 | 20 | 0 | 0 | 0 |
| Lambsquarters | 100 | 70 | 50 | 30 | 0 |
| Persian speedwell | 50 | 30 | 0 | 0 | 0 |
| Sugar beet | 60 | 20 | 0 | 0 | 0 |
| Viola | 70 | 50 | 0 | 0 | 0 |
| Wheat | 70 | 70 | 50 | 50 | 40 |
| Wild buckwheat | 60 | 20 | 20 | 20 | 0 |
| Wild oat | 40 | 30 | 0 | 0 | 0 |
| PREEMERGENCE | | | | | |
| Blackgrass | 70 | 50 | 50 | 50 | 0 |
| Catchweed bedstraw | 30 | 0 | 0 | 0 | 0 |
| Chickweed | 0 | 0 | 0 | 0 | 0 |
| Lambsquarters | 70 | - | - | 0 | 0 |
| Persian speedwell | 0 | 0 | 0 | 0 | 0 |
| Scentless chamomille | 70 | 70 | 60 | 50 | 20 |
| Sugar beet | 0 | 0 | 0 | 0 | 0 |
| Viola | 0 | 0 | 0 | 0 | 0 |
| Wheat | 40 | 30 | 0 | 0 | 0 |
| Wild buckwheat | 0 | 0 | 0 | 0 | 0 |
| Wild oat | 0 | 0 | 0 | 0 | 0 |

Table C

| | Cmpd 13 | | | | |
|---|---|---|---|---|---|
| RATE (g/ha) | 125 | 64 | 32 | 16 | 8 |
| POSTEMERGENCE | | | | | |
| Blackgrass | 100 | 100 | 90 | 80 | 50 |
| Catchweed bedstraw | 90 | 90 | 50 | 40 | 0 |
| Chickweed | 100 | 100 | 100 | 90 | 70 |
| Lambsquarters | 100 | 100 | 100 | 100 | 100 |
| Persian speedwell | 30 | 0 | 0 | 0 | 0 |
| Sugar beet | 60 | 30 | 10 | 0 | 0 |
| Viola | 80 | 80 | 40 | 0 | 0 |
| Wheat | 100 | 100 | 60 | 50 | 40 |
| Wild buckwheat | 90 | 80 | 70 | 50 | 30 |
| Wild oat | 80 | 50 | 40 | 30 | 0 |
| PREEMERGENCE | | | | | |
| Blackgrass | 90 | 90 | 80 | 30 | 20 |
| Catchweed bedstraw | 100 | 80 | 80 | 70 | 60 |
| Chickweed | 100 | 20 | 0 | 0 | 0 |
| Lambsquarters | 90 | 30 | 30 | 20 | 0 |
| Persian speedwell | 50 | 30 | 20 | 0 | 0 |
| Scentless chamomille | 90 | 90 | 90 | 90 | 80 |
| Sugar beet | 0 | 0 | 0 | 0 | 0 |
| Viola | 90 | 80 | - | - | 70 |
| Wheat | 80 | 50 | 50 | 30 | 30 |
| Wild buckwheat | 50 | 40 | 30 | 20 | 0 |
| Wild oat | 50 | 40 | 30 | 30 | 30 |

Table C

Cmpd 14

| RATE (g/ha) | 125 | 64 | 32 | 16 | 8 |
|---|---|---|---|---|---|
| POSTEMERGENCE | | | | | |
| Blackgrass | 50 | 50 | 50 | 30 | 20 |
| Catchweed bedstraw | 30 | 0 | 0 | 0 | 0 |
| Chickweed | 60 | 50 | 50 | 0 | 0 |
| Lambsquarters | 80 | 80 | 70 | 30 | 30 |
| Persian speedwell | 20 | 0 | 0 | 0 | 0 |
| Sugar beet | 30 | 30 | 20 | 0 | 0 |
| Viola | 0 | 0 | 0 | 0 | 0 |
| Wheat | 50 | 50 | 50 | 50 | 40 |
| Wild buckwheat | 70 | 50 | 50 | 20 | 20 |
| Wild oat | 60 | 60 | 60 | 50 | 50 |
| RATE (g/ha) | 250 | 125 | 64 | 32 | 16 |
| PREEMERGENCE | | | | | |
| Blackgrass | 70 | 30 | 0 | 0 | 0 |
| Catchweed bedstraw | 30 | 30 | 0 | 0 | 0 |
| Chickweed | 30 | 30 | 0 | 0 | 0 |
| Lambsquarters | 100 | 30 | 30 | 0 | 0 |
| Persian speedwell | 0 | 0 | 0 | 0 | 0 |
| Scentless chamomille | 80 | 80 | 80 | 60 | 60 |
| Sugar beet | 0 | 0 | 0 | 0 | 0 |
| Viola | 0 | 0 | 0 | 0 | 0 |
| Wheat | 0 | 0 | 0 | 0 | 0 |
| Wild buckwheat | 0 | 0 | 0 | 0 | 0 |
| Wild oat | 50 | 40 | 0 | 0 | 0 |

TEST D

Seeds selected from crop and weed species consisting of barley (Hordeum vulgare), blackgrass (Alopecurus myosuroides), bluegrass (Poa annua), chickweed (Stellaria media), field pennycress (Thlaspi arvense), green foxtail (Setaria viridis), Italian ryegrass (Lolium multiflorum), mustard (Brassica spp.), Persian speedwell (Veronica persica), pigweed (Amaranthus retroflexus), rape (Brassica napus), sugar beet (Beta vulgaris), wheat (Triticum aestivum), wild buckwheat (Polygonum convolvulus), wild oat (Avena fatua), and wild radish (Raphanus raphanistrum) were planted and treated preemergence with test chemicals dissolved in a non-phytotoxic solvent. Selected species from this list of crops and weeds were also treated with postemergence applications of test chemicals. Plants ranged in height from two to twenty cm (two to three leaf stage) for postemergence treatments. Treated plants and controls were maintained in a greenhouse for approximately 24 days, after which all species were compared to controls and visually evaluated. Response ratings, summarized in Table D, are based on a scale of 0 to 100 where 0 is no effect and 100 is complete control.

Table D

| | Cmpd 1 | |
|---|---|---|
| RATE (g/ha) | 32 | 16 |
| POSTEMERGENCE | | |
| Barley (var. "Bonanza") | 90 | 80 |
| Barley (var. "Harrington") | 90 | 80 |
| Barley (var. "Morex") | 80 | 80 |
| Blackgrass | 90 | 60 |
| Bluegrass | 70 | 30 |
| Chickweed | 80 | 80 |
| Field pennycress | 90 | 80 |
| Green foxtail | 80 | 60 |
| Italian ryegrass | 80 | 70 |
| Mustard | 80 | 60 |
| Persian speedwell | 40 | 0 |
| Pigweed | 90 | 80 |
| Rape | 0 | 0 |
| Wheat (var. "Centurk") | 90 | 90 |
| Wheat (var. "Katepwa") | 90 | 80 |
| Wild buckwheat | 40 | 40 |
| Wild oat | 90 | 80 |
| Wild radish | 60 | 50 |

| | Cmpd 2 | |
|---|---|---|
| RATE (g/ha) | 32 | 16 |
| POSTEMERGENCE | | |
| Barley (var. "Morex") | 30 | 0 |
| Chickweed | 0 | 0 |
| Green foxtail | 80 | 70 |
| Italian ryegrass | 40 | 0 |
| Mustard | 70 | 50 |
| Rape | 0 | 0 |
| Sugar beet | 0 | 0 |
| Wheat (var. "ERA") | 30 | 0 |
| Wild oat | 40 | 0 |
| Wild radish | 0 | 0 |

TABLE D

| | Cmpd 3 | |
|---|---|---|
| RATE (g/ha) | 32 | 16 |
| POSTEMERGENCE | | |
| Barley (var. "Morex") | 30 | 0 |
| Chickweed | 90 | 60 |
| Green foxtail | 70 | 0 |
| Italian ryegrass | 40 | 0 |
| Mustard | 70 | 40 |
| Rape | 0 | 0 |
| Sugar beet | 0 | 0 |
| Wheat (var. "ERA") | 60 | 40 |
| Wild oat | 0 | 0 |
| Wild radish | 0 | 0 |

| | Cmpd 10 | |
|---|---|---|
| RATE (g/ha) | 32 | 16 |
| POSTEMERGENCE | | |
| Barley (var. "Morex") | 100 | 70 |
| Chickweed | 100 | 80 |
| Green foxtail | 90 | 70 |
| Italian ryegrass | 100 | 80 |
| Mustard | 100 | 100 |
| Rape | 0 | 0 |
| Wheat (var. "ERA") | 100 | 100 |
| Wild oat | 70 | 0 |
| Wild radish | 0 | 0 |
| PREEMERGENCE | | |
| Barley (var. "Morex") | 0 | 0 |
| Chickweed | 80 | 0 |
| Green foxtail | 60 | 0 |
| Italian ryegrass | 50 | 0 |
| Mustard | 90 | 0 |
| Rape | 0 | 0 |
| Wheat (var. "ERA") | 0 | 0 |
| Wild oat | 0 | 0 |
| Wild radish | 0 | 0 |

TEST E

Seeds of alfalfa (Medicago sativa), barley (Hordeum vulgare), bluegrass (Poa pratensis), corn (Zea mays), cotton (Gossypium hirsutum), flax (Linum usitatissimum), oat (Avena sativa), pea (Pisum sativum), peanut (Arachis hypogaea), rape (Brassica napus), rice (Oryza sativa), sorghum (Sorghum bicolor), soybean (Glycine max), sugar beat (Beta vulgaris), sunflower (Helianthus annuus), tomato (Lycopersicon esculentum), and wheat (Triticum aestivum) were planted and treated with preemergence and/or postemergence applications of test chemical dissolved in a non-phytotoxic solvent. Plants ranged in height from four to twenty cm (two to three leaf stage) when post-emergence applications were applied. Treated plants and controls were grown under greenhouse conditions for approximately twenty-four days, after which all plants treated with the test chemical were compared to untreated controls and visually evaluated for injury response. Application rates for the test chemical are shown in Table E. Plant response ratings, summarized in Table E, are from 0 to 100 where 0 is no injury and 100 is complete control.

Table E

| Cmpd 1 | | | | | |
|---|---|---|---|---|---|
| RATE (g/ha) | 125 | 64 | 32 | 16 | 8 |
| POSTEMERGENCE | | | | | |
| Alfalfa | 95 | 90 | 60 | 40 | 25 |
| Barley | 100 | 80 | 55 | 25 | 0 |
| Bluegrass | 100 | 100 | 85 | 50 | 30 |
| Corn | 100 | 100 | 100 | 80 | 75 |
| Cotton | 95 | 85 | 65 | 40 | 20 |
| Oat | 100 | 100 | 65 | 30 | 0 |
| Pea | 100 | 95 | 90 | 55 | 35 |
| Peanut | 95 | 90 | 80 | 60 | 35 |
| Rape | 30 | 20 | 0 | 0 | 0 |
| Rice | 100 | 95 | 80 | 55 | 40 |
| Sorghum | 100 | 100 | 85 | 65 | 30 |
| Soybean | 100 | 95 | 80 | 50 | 35 |
| Sugar beet | 40 | 35 | 20 | 0 | 0 |
| Tomato | 85 | 80 | 60 | 40 | 20 |
| Wheat | 95 | 70 | 50 | 25 | 0 |
| RATE (g/ha) | 250 | 125 | 64 | 32 | 16 |
| PREEMERGENCE | | | | | |
| Alfalfa | 100 | 95 | 60 | 45 | 20 |
| Barley | 100 | 75 | 50 | 35 | 20 |
| Bluegrass | 100 | 100 | 90 | 70 | 25 |
| Corn | 100 | 95 | 75 | 40 | 0 |
| Cotton | 65 | 30 | 0 | 0 | 0 |
| Flax | 30 | 0 | 0 | 0 | 0 |
| Oat | 100 | 100 | 80 | 70 | 40 |
| Pea | 90 | 65 | 40 | 20 | 0 |
| Peanut | 80 | 65 | 55 | 35 | 0 |
| Rape | 20 | 0 | 0 | 0 | 0 |
| Rice | 100 | 95 | 75 | 40 | 0 |
| Sorghum | 100 | 100 | 100 | 75 | 30 |
| Soybean | 70 | 60 | 45 | 20 | 0 |
| Sugar beet | 20 | 0 | 0 | 0 | 0 |
| Sunflower | 95 | 80 | 70 | 45 | 20 |
| Tomato | 85 | 55 | 40 | 0 | 0 |
| Wheat | 100 | 95 | 65 | 40 | 25 |

TEST F

Seeds of flax (Linum usitatissimum), lentil (Lens culinaris), safflower (Carthamus tinctorius), and wild mustard (Brassica kaber) were planted into greenhouse potting medium. When the plants were in the 2 to 4 leaf stage, they were treated with postemergence applications of test chemical dissolved in a non-phytotoxic solvent. Treated plants and controls were grown in the greenhouse for 14 days before they were visually evaluated for injury response. Response ratings, summarized in Table F, are from zero to 100 where zero is no injury, 10 is minimal injury, and 100 is plant death. Note the herbicidal activity on the troublesome weed, wild mustard, with crop safety to flax, lentil, and safflower.

Table F

| | Cmpd 1 | | |
|---|---|---|---|
| RATE (g/ha) | 125 | 64 | 32 |
| POSTEMERGENCE | | | |
| Flax | 0 | 0 | 0 |
| Lentil | 0 | 0 | 0 |
| Safflower | 0 | 0 | 0 |
| Wild mustard | 100 | 100 | 100 |

TEST G

Seeds of annual bluegrass (Poa annua), barley (Hordeum vulgare var. "Morex"), black nightshade (Solanum nigrum), blackgrass (Alopecurus myosuroides), broccoli (Brassica oleracea var. "Botrytis"), cabbage (Brassica oleracea var. "Capitata"), catchweed bedstraw (Galium aparine), cauliflower (Brassica oleracea var. "Botrytis"), chickweed (Stellaria media), crambe (Capsella abyssinica), field violet (Viola arvensis), green foxtail (Setaria viridis), Italian ryegrass (Lolium multiflorum), lambsquarters (Chenopodium album), lettuce (Lactuca sativa), Persian speedwell (Veronica persica), rape (Brassica napus var. "Westar" and "Bienvenu"), scentless chamomille (Matricaria inodora), shepherd's purse (Capsella bursa-pastoris), smartweed (Polygonum pensylvanicum), stinkweed (Thlaspi arvense), wheat (Triticum aestivum var. "Park"), wild buckwheat (Polygonum convolvulus), wild mustard (Brassica kaber), wild oat (Avena fatua), and wild radish (Raphanus raphanistrum) were planted in greenhouse potting medium. When plants were in the 2 to 4 leaf stage, they were treated with postemergence applications of test chemical dissolved in a non-phytotoxic solvent. Treated plants and controls were grown in the greenhouse and visually evaluated for injury response 18 days after treatment. Plant response ratings, summarized in Table G, are from zero to 100 where zero is no injury, 10 is minimal injury, and 100 is plant death.

Table G

| | Cmpd 1 | | |
|---|---|---|---|
| RATE (g/ha) | 32 | 16 | 8 |
| POSTEMERGENCE | | | |
| Annual bluegrass | 60 | 60 | 40 |
| Barley (var. "Morex") | 60 | 60 | 50 |
| Black nightshade | 0 | 0 | 0 |
| Blackgrass | 70 | 70 | 30 |
| Broccoli | 0 | 0 | 0 |
| Cabbage | 0 | 0 | 0 |
| Catchweed bedstraw | 100 | 100 | 75 |
| Cauliflower | 0 | 0 | 0 |
| Chickweed | 95 | 75 | 55 |
| Crambe | 20 | 0 | 0 |
| Field violet | 85 | 60 | 50 |
| Green foxtail | 55 | 50 | 30 |
| Italian ryegrass | 60 | 40 | 0 |
| Lambsquarters | 30 | 20 | 0 |
| Lettuce | 85 | 45 | 45 |
| Persian speedwell | 100 | 50 | 0 |
| Rape (var. "Bienvenu") | 0 | 0 | 0 |
| Rape (var. "Westar") | 0 | 0 | 0 |
| Scentless chamo-mille | 100 | 0 | 0 |
| Shep-herd's purse | 100 | 100 | 80 |
| Smartweed | 65 | 55 | 30 |
| Stinkweed | 100 | 100 | 60 |
| Wheat (var. "Park") | 70 | 60 | 50 |
| Wild buckwheat | 60 | 30 | 15 |
| Wild mustard | 70 | 60 | 50 |
| Wild oat | 75 | 60 | 30 |
| Wild radish | 35 | 0 | 0 |

TEST H

Seeds of alfalfa (Medicago sativa), ball mustard (Neslia paniculata), barley (Hordeum vulgare var. "Bonanza" and "Morex"), black mustard (Brassica nigra), black nightshade (Solanum nigrum), broadleaf plantain (Plantago major), brown mustard (Brassica juncea), buckhorn plantain (Plantago lanceolata), dandelion (Taraxacum officinale), field bean (Phaseolus spp.), field violet (Viola arvensis), flax (Linum usitatissimum), groundsel (Senecio vulgaris), Kentucky bluegrass (Poa pratensis), kochia (Kochia scoparia), lentil (Lens culinaris), pea (Pisum sativum), peanut (Arachis hypogaea), rape (Brassica napus var. "Westar") red clover

(Trifolium pratense), Russian thistle (Salsola kali), safflower (Carthamus tinctorius), scentless chamomille (Matricaria inodora), shepherd's purse (Capsella bursa-pastoris), sowthistle (Sonchus oleraceus), sugar beet (Beta vulgaris), sunflower (Helianthus annuus), tansy mustard (Descurainia pinnata), vetch (Vicia sativa), wheat (Triticum aestivum var. "Centurk"), wild mustard (Brassica kaber), wild radish (Raphanus raphanistrum), and yellow mustard (Brassica hirta) were planted into greenhouse potting medium. When plants were in the 2 to 4 leaf stage, they were treated with postemergence applications of test chemical dissolved in a non-phytotoxic solvent. Treated plants and controls were grown in the greenhouse for 18 days before they were visually evaluated for response injury. Injury responses, summarized in Table H, are from zero to 100 where zero is no injury, 10 is minimal injury, and 100 is plant death. A dash (-) response means no test result.

Table H

| | Cmpd 1 | | |
|---|---|---|---|
| RATE (g/ha) | 32 | 16 | 8 |
| POSTEMERGENCE | | | |
| Alfalfa | 60 | 30 | 0 |
| Ball mustard | 100 | 90 | - |
| Barley (var. "Bonanza") | 70 | 50 | 20 |
| Barley (var. "Morex") | 80 | 40 | 20 |
| Black mustard | 0 | 0 | - |
| Black nightshade | 0 | 0 | 0 |
| Broadleaf plantain | 0 | 0 | 0 |
| Brown mustard | 20 | 0 | - |
| Buckhorn plantain | 0 | 0 | 0 |
| Dandelion | 0 | 0 | 0 |
| Field bean | 50 | 40 | 20 |
| Field violet | 40 | 30 | 20 |
| Flax | 0 | 0 | 0 |
| Groundsel | 30 | 20 | 0 |
| Kentucky bluegrass | 60 | 50 | - |
| Kochia | 0 | 0 | 0 |
| Lentil | 0 | 0 | 0 |
| Pea | 100 | 100 | 100 |
| Peanut | 70 | 30 | 20 |
| Rape (var. "Westar") | 0 | 0 | - |
| Red clover | 70 | 40 | 0 |
| Russian thistle | 40 | 30 | 0 |
| Safflower | 20 | 0 | 0 |
| Scentless chamomille | 80 | 20 | 0 |
| Shepherd's purse | 60 | 20 | 0 |
| Sowthistle | 0 | 0 | 0 |
| Sugar beet | 0 | 0 | 0 |
| Sunflower | 90 | 90 | 90 |
| Tansy mustard | 100 | 90 | - |
| Vetch | 0 | 0 | 0 |
| Wheat (var. "Centurk") | 80 | 70 | 60 |
| Wild mustard | 100 | 70 | - |
| Wild radish | 60 | 20 | - |
| Yellow mustard | 30 | 20 | - |

Table H

| | Cmpd 3 | | |
|---|---|---|---|
| RATE (g/ha) | 32 | 16 | 8 |
| POSTEMERGENCE | | | |
| Alfalfa | 40 | 0 | 0 |
| Ball mustard | 80 | 70 | 40 |
| Barley (var. "Bonanza") | 30 | 20 | 0 |
| Barley (var. "Morex") | 40 | 30 | 0 |
| Black mustard | 0 | 0 | 0 |
| Black nightshade | 0 | 0 | 0 |
| Broadleaf plantain | 0 | 0 | 0 |
| Brown mustard | 0 | 0 | 0 |
| Buckhorn plantain | 0 | 0 | 0 |
| Dandelion | 40 | 20 | 0 |
| Field bean | 50 | 20 | 20 |
| Field violet | 20 | 20 | 0 |
| Flax | 0 | 0 | 0 |
| Groundsel | 0 | 0 | 0 |
| Kentucky bluegrass | 0 | 0 | 0 |
| Kochia | 0 | 0 | 0 |
| Lentil | 0 | 0 | 0 |
| Pea | 40 | 20 | 0 |
| Peanut | 20 | 0 | 0 |
| Rape (var. "Westar") | 0 | 0 | 0 |
| Red clover | 60 | 20 | 0 |
| Russian thistle | 0 | 0 | 0 |
| Safflower | 30 | 10 | 0 |
| Scentless chamomille | 60 | 30 | 20 |
| Shepherd's purse | 40 | 30 | 0 |
| Sowthistle | 0 | 0 | 0 |
| Sugar beet | 0 | 0 | 0 |
| Sunflower | 80 | 80 | 60 |
| Tansy mustard | 70 | 70 | 30 |
| Vetch | 0 | 0 | 0 |
| Wheat (var. "Centurk") | 50 | 40 | 20 |
| Wild mustard | 100 | 80 | 50 |
| Wild radish | 50 | 0 | 0 |
| Yellow mustard | 0 | 0 | 0 |

TEST I

Seeds of broccoli (Brassica oleracea var. "Botrytis"), brussel sprouts (Brassica oleracea var. "Gemmifera"), cabbage (Brassica oleracea var. "Capitata"), cauliflower (Brassica oleracea var. "Botrytis"), crambe (Crambe abyssinica), kale (Brassica oleracea var. "Acephala"), kohlrabi (Brassica oleracea var. "Canlorapa"), mustard (Brassica juncea), rape (Brassica napus var. "Westar"), and turnip (Brassica rapa) were planted into a greenhouse potting soil. When plants were in the 2 to 3 leaf stage, they were treated with postemergence applications of test chemical dissolved in a non-phytotoxic solvent. Treated plants and controls were grown in the greenhouse for 23 days before visual evaluations of plant injury responses were recorded. Response ratings, summarized in Table I, are from zero to 100 where zero is no injury, 10 is minimal injury, and 100 is plant death. Note in particular the low herbicidal activity of Brassica ssp. crops (cole crops).

Table I

| Cmpd 1 RATE (g/ha) | 32 | 16 | 8 |
|---|---|---|---|
| POSTEMERGENCE | | | |
| Broccoli | 0 | 0 | 0 |
| Brussel sprouts | 0 | 0 | 0 |
| Cabbage | 0 | 0 | 0 |
| Cauliflower | 0 | 0 | 0 |
| Crambe | 0 | 0 | 0 |
| Kale | 0 | 0 | 0 |
| Kohlrabi | 0 | 0 | 0 |
| Mustard | 0 | 0 | 0 |
| Rape (var. "Westar") | 0 | 0 | 0 |
| Turnip | 0 | 0 | 0 |

TEST J

Seeds of brussel sprouts (Brassica oleracea var. "Long Island Improved"), cabbage (Brassica oleracea var. "Premium Flat Dutch"), cauliflower (Brassica oleracea var. "Burpeeane"), kohlrabi (Brassica oleracea var. "Early White Vienna"), pak choi (Brassica rapus var. "Lei Choy"), red beet (Beta maritima var. "Ruby Queen"), and sugar beet (Beta vulgaris var. "Kawemono") were grown in a sandy loam soil under greenhouse conditions. These plant species were treated with preemergence and postemergence applications of test chemical dissolved in a non-phytotoxic solvent. For preemergence treatments, the compound was applied immediately after the seeds were planted, while, for postemergence applications, the chemical was applied when plants were in the 2 to 3 leaf stage (approximately 12 days old). Treated plants and controls were grown in the greenhouse for 21 days before visual evaluations of injury responses were recorded. Plant response ratings, summarized in Table J, are from zero to 100 where zero is no injury, 10 is minimal injury, and 100 is plant death. Note in particular the tolerance of these crops to the test chemical.

Table J

| Cmpd 1 RATE (g/ha) | 250 | 125 | 64 | 32 | 16 | 8 |
|---|---|---|---|---|---|---|
| POSTEMERGENCE | | | | | | |
| Brussel sprouts | 20 | 20 | 0 | 0 | 0 | 0 |
| Cabbage | 10 | 10 | 0 | 0 | 0 | 0 |
| Cauliflower | 30 | 10 | 0 | 0 | 0 | 0 |
| Kohlrabi | 20 | 0 | 0 | 0 | 0 | 0 |
| Pak choi | 40 | 30 | 20 | 20 | 10 | 0 |
| Red beet | 30 | 20 | 10 | 10 | 0 | 0 |
| Sugar beet | 20 | 20 | 20 | 10 | 0 | 0 |
| PREEMERGENCE | | | | | | |
| Brussel sprouts | 20 | 20 | 0 | 0 | 0 | 0 |
| Cabbage | 0 | 0 | 0 | 0 | 0 | 0 |
| Cauliflower | 60 | 30 | 30 | 10 | 0 | 0 |
| Kohlrabi | 30 | 0 | 0 | 0 | 0 | 0 |
| Pak choi | 30 | 10 | 10 | 0 | 0 | 0 |
| Red beet | 0 | 0 | 0 | 0 | 0 | 0 |
| Sugar beet | 20 | 0 | 0 | 0 | 0 | 0 |

## Claims

1. A method for controlling the growth of undesired vegetation in crops which comprises applying to the locus to be protected an effective amount of a compound of the formula

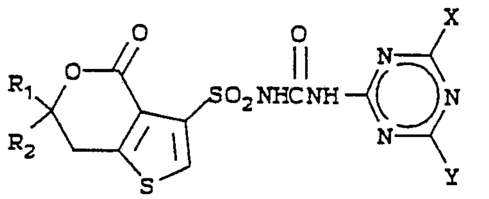

I

whereinR1 is H, $CH_3$ or $CH_2CH_3$;
$R_2$ is H or $CH_3$;
X is $OCH_3$ or $OCH_2CH_3$; and
Y is $NHCH_3$, $N(CH_3)_2$, $N(CH_3)OCH_3$, $OCH_3$, $OCH_2CH_3$, $OCH_2CF_3$, $CH_2CH_3$, $CH(CH_3)_2$ or cyclopropyl; and their agriculturally suitable salts.

2. The method of Claim 1 wherein $R_1$ and $R_2$ in the compound are hydrogen.

3. The method of Claim 1 wherein the compound is:
N-[[[4-(dimethylamino)-6-ethoxy-1,3,5-triazin-2-yl]amino]carbonyl]-6,7-dihydro-4-oxo-4H-thieno[3,2-c]pyran-3-sulfonamide.

4. The method of either Claims 1, 2 or 3 wherein the crop is selected from the group consisting of sugar beet, oilseed rape, cole, lentil, flax and safflower.

5. The method of Claim 4 wherein the crop is sugar beet.

6. A compound of the formula

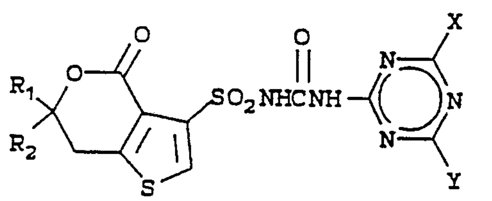

I

wherein
$R_1$ is H, $CH_3$ or $CH_2CH_3$;
$R_2$ is H or $CH_3$;
X is $OCH_3$ or $OCH_2CH_3$; and
Y is $NHCH_3$, $N(CH_3)_2$, $N(CH_3)OCH_3$, $OCH_3$, $OCH_2CH_3$, $OCH_2CF_3$, $CH_2CH_3$, $CH(CH_3)_2$ or cyclopropyl;
provided however that when $R_1$ and $R_2$ are H and Y is $NHCH_3$, then X is $OCH_3$;
and their agriculturally suitable salts.

7. The compound of Claim 6 wherein $R_1$ and $R_2$ are H.

8. The compound of Claim 1 which is:
N-[[[4-(dimethylamino)-6-ethoxy-1,3,5-triazin-2-yl]amino]carbonyl]-6,7-dihydro-4-oxo-4H-thieno[3,2-c]pyran-3-sulfonamide.

9. A composition suitable for controlling the growth of undesired vegetation which comprises an effective amount of a compound of either Claims 6, 7 or 8 and at least one of the following: surfactant, solid diluent or liquid diluent.